# EUROPEAN PATENT SPECIFICATION

(11) **EP 2 401 955 B1**
(45) Date of publication and mention of the grant of the patent: **02.11.2016**
(21) Application number: 11171662.7
(22) Date of filing: 28.06.2011
(51) Int. Cl.: A61B 1/12, A61B 1/015, A61B 1/00

(54) **DISTAL END PART FOR SUPPLYING GAS AND LIQUID IN AN ENDOSCOPE**
TEIL ZUR GAS- UND FLÜSSIGKEITSVERSORGUNG FÜR DAS DISTALE ENDE EINES ENDOSKOPS
DISTALE PARTIE FIN POUR ALIMENTER EN GAZ ET LIQUIDE DANS UN ENDOSCOPE

(30) Priority: 29.06.2010 JP 2010147505; 13.07.2010 JP 2010159004
(43) Date of publication of application: 04.01.2012
(60) Divisional application: 13169744.3
(73) Proprietor: Fujifilm Corporation, Minato-ku Tokyo 106-8620 (JP)
(72) Inventor: Naito, Kan, Ashigarakami-gun Kanagawa, 258-8538 (JP); Ikeda, Toshiyuki, Ashigarakami-gun Kanagawa, 258-8538 (JP)
(74) Representative: Klunker . Schmitt-Nilson . Hirsch

(56) References cited:
- DE-A1- 3 004 089
- DE-A1-102004 029 099
- JP-A- 2007 229 261
- JP-A- 2008 086 649
- JP-B2- 3 017 754
- US-A- 5 685 823
- US-A- 5 840 016
- US-A1- 2002 040 181
- US-A1- 2009 253 965

## Description

### BACKGROUND OF THE INVENTION

### Field of the Invention

The presently disclosed subject matter relates to a distal end part for an endoscope and more particularly, to a structure of a gas supply pipe which is a flow channel of air (gas) and a structure of a liquid supply pipe which is a flow channel of a cleaning liquid, the air (gas) and the cleaning liquid being used for cleaning an observation window and the like of an endoscope.

### Description of the Related Art

An endoscope for medical use and the like is provided with an illumination window and an observation window at the distal end of an insertion part which is inserted into a body, and the inside of the body is observed via the observation window under illumination light emitted through the illumination window. However, if a fouling substance such as a body fluid attaches to a surface of the observation window, the observation field of view inside of the body is limited, and the sharpness of an observation image obtained via the observation window is deteriorated.

Therefore, an observation window cleaning apparatus which cleans up the observation window with the insertion part being inserted into the body is provided. The observation window cleaning apparatus injects a cleaning liquid toward the observation window to wash an attached substance away, and then injects a pressurized gas, to thereby remove liquid drops attached to the surface of the observation window. Normally, water is used as the cleaning liquid, and air is used as the pressurized gas.

A configuration example of the observation window cleaning apparatus includes a configuration including: an injection nozzle provided in the vicinity of the observation window; and a gas supply and liquid supply apparatus for supplying the cleaning liquid and air to the injection nozzle. In general, the gas supply and liquid supply apparatus includes: a cleaning liquid pipe line and an air pipe line connected to the injection nozzle; a gas supply and liquid supply valve which makes switching between three states of a fluid supply stop state, a gas supply state, and a liquid supply state; and an operation button for performing such control that the cleaning liquid or pressurized air is supplied to the injection nozzle via the cleaning liquid pipe line and the air pipe line, the operation button being provided in a main body operation part. In the observation window cleaning apparatus having such a configuration, the operation button provided in the main body operation part is operated by an operator, whereby the switching of the gas supply and liquid supply valve is performed.

With regard to a gas supply and liquid supply apparatus applicable to the observation window cleaning apparatus, Japanese Patent No. 3678614 discloses a structure in which a pipe line bent into an L shape is coupled to a middle of a pipe line provided in a linear fashion (see Fig. 6 in Japanese Patent No. 3678614).

Japanese Patent Application Laid-Open No. 2007-236425 discloses a flow channel confluence structure including a flow channel confluence part which is formed so as to become gradually wider from a downstream-side flow channel attachment hole toward an upstream-side flow channel attachment hole (see Fig. 3 in Japanese Patent Application Laid-Open No. 2007-236425).

Japanese Patent Application Laid-Open No. 2009-279299 discloses a gas supply and liquid supply pipe line including a metal pipe part having a curved (bent) shape and a flexible pipe part (see Fig. 2 in Japanese Patent Application Laid-Open No. 2009-279299). One end of the metal pipe part is formed as a gas supply and liquid supply port, and the gas supply and liquid supply port is connected to a tank.

Japanese Patent Application Laid-Open No. 2007-185387 discloses a structure in which a gas supply tube and a liquid supply tube are connected to a gas supply and liquid supply connector via a gas supply and liquid supply ferrule (see Figs. 1 and 4 in Japanese Patent Application Laid-Open No. 2007-185387).

Japanese Patent Application Laid-Open No. 2000-287974 discloses a piping structure of an endoscope in which a gas supply tube and a liquid supply tube are brought together to be connected into one pipe line by a first bifurcation connection member (see Fig. 2 in Japanese Patent Application Laid-Open No. 2000-287974).

US 5,685,823 discloses an distal end part of an endoscope comprising a confluence pipe. An endoscope of interest is also disclosed in JP 2007-229261.

### SUMMARY OF THE INVENTION

However, inside of the insertion part (distal end part) in which the gas supply pipe line and the liquid supply (water supply) pipe line are provided, an image pick-up unit for picking up an image of an observation subject via the observation window, a component for angling the distal end part to change the orientation thereof, and the like are provided, and hence convexo-concaves exist inside of the insertion part (distal end part).

On the other hand, there are positions best suited to the gas supply pipe line (gas supply tube) and the liquid supply pipe line (liquid supply tube), but the existence of the convexo-concaves sometimes makes it difficult to place the gas supply pipe line and the liquid supply pipe line at the best suited positions. If the gas supply pipe line and the liquid supply pipe line are placed at the best suited positions while avoiding the convexo-concaves inside of the distal end part, the size in the radial direction and the size in the longitudinal direction of the distal end part become larger.

In addition, if the gas supply tube as the gas supply pipe line and the liquid supply tube as the liquid supply pipe line are placed so as to avoid a cable connected to the image pick-up unit, other tubes, and the like, a portion which is subject to a stress caused by bending may locally exist. In this case, the durability of the gas supply tube and the liquid supply tube becomes problematic.

Meanwhile, in the confluence structure according to the related art in which the gas supply tube and the liquid supply tube are brought together, a portion which is communicated with the gas supply tube and the liquid supply tube doubles as a confluence part as in a substantial Y shape and the bifurcation connection member (piping structure) disclosed in Japanese Patent Application Laid-Open No. 2000-287974. In this case, the placement of the gas supply tube and the liquid supply tube which are communicated with the confluence part is determined by the structure and shape of the confluence part, and hence it is extremely difficult to place the gas supply tube and the liquid supply tube at the best suited positions.

The presently disclosed subject matter has been made in view of the above-mentioned circumstances, and therefore has an object to provide a gas supply and liquid supply apparatus for an endoscope which is capable of realizing preferable placement of a gas supply pipe line (gas supply tube) and a liquid supply pipe line (liquid supply tube), avoiding an increase in size of an insertion part (distal end part), and securing predetermined durability of the gas supply tube and the liquid supply tube.

In order to achieve the above-mentioned object, a distal end part for an endoscope according to claim 1 is the invention. It includes: a first fluid pipe which is provided in a distal end part of an insertion part of an endoscope, the first fluid pipe for supplying a first fluid; a second fluid pipe which is provided in the distal end part of the insertion part of the endoscope together with the first fluid pipe, the second fluid pipe for supplying a second fluid; and a confluence pipe which is connected to the first fluid pipe and the second fluid pipe, a central axis of the confluence pipe in a connection portion to the first fluid pipe and the second fluid pipe having a skew relationship to at least any one of a central axis of the first fluid pipe and a central axis of the second fluid pipe.

According to the presently disclosed subject matter, in a confluence part of the confluence pipe between the first fluid pipe and the second fluid pipe, the central axis of the confluence pipe has the skew relationship to at least any one of the central axis of the first fluid pipe and the central axis of the second fluid pipe. Accordingly, the first fluid pipe and the second fluid pipe which are connected to the confluence pipe can be three-dimensionally placed so as to suit a surrounding structure, and an enhancement in space efficiency of the portion in which the first fluid pipe and the second fluid pipe are placed can be expected.

### BRIEF DESCRIPTION OF THE DRAWINGS

Fig. 1 is an overall configuration view illustrating an endoscope according to a first embodiment of the presently disclosed subject matter;
Fig. 2 is a plan view illustrating a configuration of a distal end surface illustrated in Fig. 1;
Fig. 3 is a cross sectional view illustrating a three-dimensional structure of a distal end part illustrated in Fig. 1;
Fig. 4 is a plan view illustrating a confluence pipe illustrated in Fig. 3;
Fig. 5 is a cross sectional view taken along the 5-5 line illustrated in Fig. 4;
Fig. 6 is a cross sectional view taken along the 6-6 line illustrated in Fig. 4;
Fig. 7 is a cross sectional view taken along the 7-7 line illustrated in Fig. 4;
Fig. 8 is a see-through perspective view illustrating a schematic structure of the distal end part illustrated in Fig. 1;
Fig. 9 is a view for describing a method of fixing tubes with a double lumen structure, the method being applied to a gas supply tube and a liquid supply tube;
Fig. 10A is a perspective view illustrating a joining member which is used for fixing the tubes with the double lumen structure, Fig. 10B is an explanatory view illustrating a state where the joining member illustrated in Fig. 10A is attached to the gas supply tube and the liquid supply tube, and Fig. 10C is an explanatory view schematically illustrating another method of fixing the tubes with the double lumen structure illustrated in Fig. 9;
Fig. 11 is a view for describing still another method of fixing the tubes with the double lumen structure illustrated in Fig. 9;
Fig. 12A is a plan view illustrating a schematic structure of a bending part, and Fig. 12B is an explanatory view schematically illustrating a fixing structure for wires of the bending part;
Fig. 13 is a view for describing a first modified example of the layout of the gas supply pipe and the liquid supply pipe;
Fig. 14 is a view for describing a second modified example of the layout of the gas supply pipe and the liquid supply pipe;
Fig. 15 is a view for describing a third modified example of the layout of the gas supply pipe and the liquid supply pipe;
Fig. 16 is a view for describing a fourth modified example of the layout of the gas supply pipe and the liquid supply pipe;
Fig. 17 is a view for describing a fifth modified example of the layout of the gas supply pipe and the liquid supply pipe;
Fig. 18A is a plan view illustrating a confluence pipe according to a sixth modified example, and Fig. 18B is a cross sectional view illustrating a schematic configuration of a distal end part according to the sixth modified example;
Fig. 19 is a cross sectional view illustrating a three-dimensional structure of a distal end part of an endoscope according to a second embodiment of the presently disclosed subject matter (a cross sectional view corresponding to the 6-6 cross sectional view illustrated in Fig. 4);
Fig. 20 is a cross sectional view illustrating a three-dimensional structure of a distal end part of an endoscope according to a third embodiment of the presently disclosed subject matter (a cross sectional view corresponding to the 6-6 cross sectional view illustrated in Fig. 4);
Fig. 21 is a cross sectional view illustrating an internal structure of a distal end part according to a fourth embodiment not being part of the invention;
Fig. 22 is a perspective view illustrating a schematic structure of a confluence pipe which is applied to an endoscope illustrated in Fig. 21;
Fig. 23 is a cross sectional view illustrating the confluence pipe illustrated in Fig. 22, which is taken along the 23-23 line;
Fig. 24 is a cross sectional view illustrating the confluence pipe illustrated in Fig. 22, which is taken along the 24-24 line;
Fig. 25 is a cross sectional view illustrating the confluence pipe illustrated in Fig. 22, which is taken along the 25-25 line;
Fig. 26 is a perspective view illustrating another mode of the confluence pipe illustrated in Fig. 22;
Fig. 27 is a see-through perspective view illustrating a schematic structure of the distal end part illustrated in Fig. 21;
Fig. 28 is a perspective view illustrating a schematic structure of a confluence pipe according to a fifth embodiment not being part of the invention;
Fig. 29A is a cross sectional view illustrating the confluence pipe illustrated in Fig. 28, and Fig. 29B is a cross sectional view taken along the 29B-29B line in Fig. 29A;
Fig. 30 is a perspective view illustrating a schematic structure of a confluence pipe according to a sixth embodiment not being part of the invention;
Fig. 31A is a cross sectional view illustrating the confluence pipe illustrated in Fig. 30, and Fig. 31B is a cross sectional view taken along the 31B-31B line in Fig. 31 A;
Fig. 32A is a cross sectional view illustrating a structure of a distal end part according to a seventh modified example not being part of the invention, and Fig. 32B is a plan view illustrating the structure of the distal end part according to the seventh modified example of the presently disclosed subject matter ;
Fig. 33 is a cross sectional view illustrating a structure of a distal end part according to an eighth modified example not being part of the invention;
Fig. 34 is a cross sectional view illustrating a structure of another mode of the distal end part illustrated in Fig. 33;
Fig. 35 is a perspective view illustrating a confluence pipe according to a ninth modified example not being part of the invention;
Fig. 36 is a perspective view illustrating another mode of the confluence pipe illustrated in Fig. 35; and
Fig. 37 is a cross sectional view illustrating a structure of a distal end part according to a tenth modified example not being part of the invention.

### DETAILED DESCRIPTION OF THE PREFERRED EMBODIMENTS

Hereinafter, preferred embodiments of the presently disclosed subject matter are described in detail with reference to the attached drawings.

### [First Embodiment]

### (Overall Configuration of Endoscope)

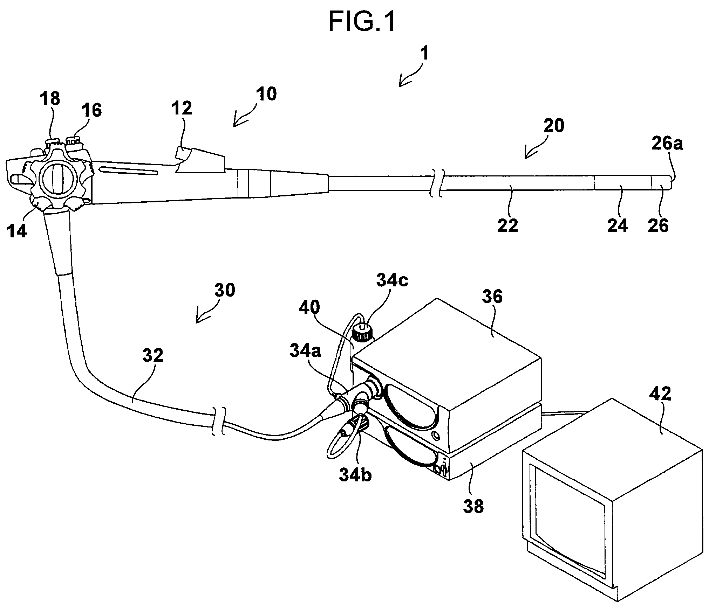

Fig. 1 is an overall configuration view illustrating a schematic structure of an endoscope according to an embodiment of the presently disclosed subject matter. An endoscope 1 illustrated in Fig. 1 is an electronic endoscope which takes out a subject image inside of a body cavity as an electronic image, and includes: an operation part 10 which is used by an operator in order to perform a required operation; an insertion part 20 which is inserted into the body cavity; and a connection part 30 for connecting with a processor apparatus and the like.

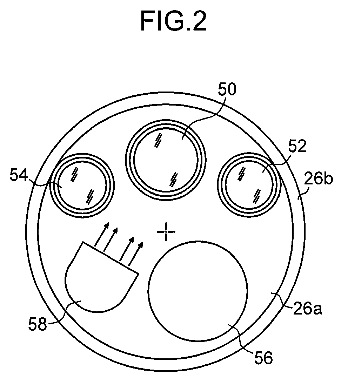

The operation part 10 includes: a forceps entrance 12 for inserting a treatment tool; an angle knob 14 for bending the distal end of the insertion part 20 up, down, right, or left; a gas supply and liquid supply button 16 for ejecting water and air or carbon dioxide gas (hereinafter, it is assumed in the present specification that the "gas" ejected from a nozzle includes at least any one of air and carbon dioxide gas.) from the nozzle (not illustrated in Fig. 1; illustrated in Fig. 2 by reference numeral 58) provided at the distal end of the insertion part 20, to thereby clean an observation window (not illustrated in Fig. 1; illustrated in Fig. 2 by reference numeral 50) provided at the distal end of the insertion part 20; and a suction button 18 for suctioning via a forceps exit (not illustrated in Fig. 1; illustrated in Fig. 2 by reference numeral 56) provided at the distal end of the insertion part 20.

The insertion part 20 is formed into a pipe-like shape which is substantially circular with a predetermined radius in cross section, and is integrally provided so as to be continuous with the distal end of the operation part 10. The insertion part 20 includes: a flexible part 22 having flexibility; a bendable bending part 24 provided at the distal end of the flexible part 22; and a distal end part (distal end hard part) 26 provided at the distal end of the bending part 24.

The flexible part 22 is configured by a flexible pipe, and is integrally provided so as to be continuous with the distal end of the operation part 10. A large part of the insertion part 20 is configured by the flexible part 22. The bending part 24 is configured to be bendable, and is integrally provided so as to be continuous with the distal end of the flexible part 22.

The bending part 24 bends up, down, right, or left so as to follow an operation on the angle knob 14 provided in the operation part 10. Accordingly, the distal end part 26 can be turned in a desired direction inside of the body cavity by bending the bending part 24 in the desired direction.

The distal end part 26 is formed into a columnar shape by using a hard material such as metal (for example, stainless), and is integrally provided so as to be continuous with the distal end of the bending part 24.

The connection part 30 includes: a universal code 32 which is provided so as to be continuous with the operation part 10; and a plurality of connectors which are provided in the distal end part of the universal code 32.

These connectors include: a processor connector 34a for connecting with a processor apparatus 36; a light source connector 34b for connecting with a light source apparatus 38; and a gas supply and liquid supply connector 34c for connecting with a gas supply and liquid supply apparatus (not illustrated) provided in a casing in which the processor apparatus 36 is installed.

### (Description of Distal end part)

Fig. 2 is a plan view illustrating a structure of a distal end surface 26a of the distal end part 26 illustrated in Fig. 1. The distal end surface 26a illustrated in Fig. 2 has a substantially circular planar shape. The distal end part 26, on the distal end surface 26a, includes: the observation window 50 which is placed at a position close to the outer circumference of the distal end surface 26a, and serves to observe a region to be observed; a pair of illumination windows 52 and 54 which is placed at positions close to the outer circumference on both sides of the observation window 50 so as to sandwich the observation window 50, and serves to illuminate the region to be observed with illumination light; the forceps exit 56 which serves as the exit of the treatment tool inserted from the forceps entrance 12 (see Fig. 1); and a nozzle 58 for jetting a cleaning liquid and air toward the observation window 50.

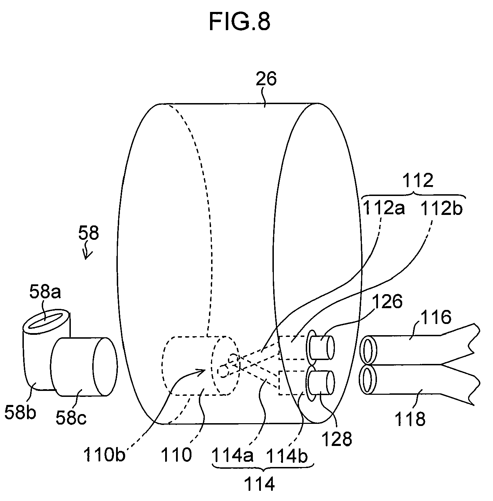

The nozzle 58 is placed so that an ejection port (not illustrated in Fig. 2; illustrated in Fig. 8 by reference numeral 58a) thereof faces the observation window 50, and the forceps exit 56 is placed adjacently to the nozzle 58. In addition, an edge part 26b at the outer circumference of the distal end surface 26a is round-chamfered at a predetermined diameter.

An illumination optical system is placed behind (on the inner side of) each of the paired illumination windows 52 and 54 illustrated in Fig. 2. A light guide (not illustrated) provided inside of the insertion part 20 illustrated in Fig. 1 is connected to the illumination optical system. When the light source connector 34b of the connection part 30 is connected to the light source apparatus 38, this light guide is connected to a light source lamp (not illustrated) incorporated in the light source apparatus 38.

Accordingly, when the light source lamp of the light source apparatus 38 is turned on, light emitted from the light source lamp is guided by the light guide to the illumination optical system. Then, the light guided to the illumination optical system illuminates the region to be observed via the illumination windows 52 and 54 illustrated in Fig. 2.

The forceps exit 56 illustrated in Fig. 2 is connected to the forceps entrance 12 of the operation part 10 via a forceps channel (not illustrated) provided inside of the insertion part 20 illustrated in Fig. 1. The treatment tool such as forceps which is inserted from the forceps entrance 12 protrudes from the forceps exit 56 illustrated in Fig. 2.

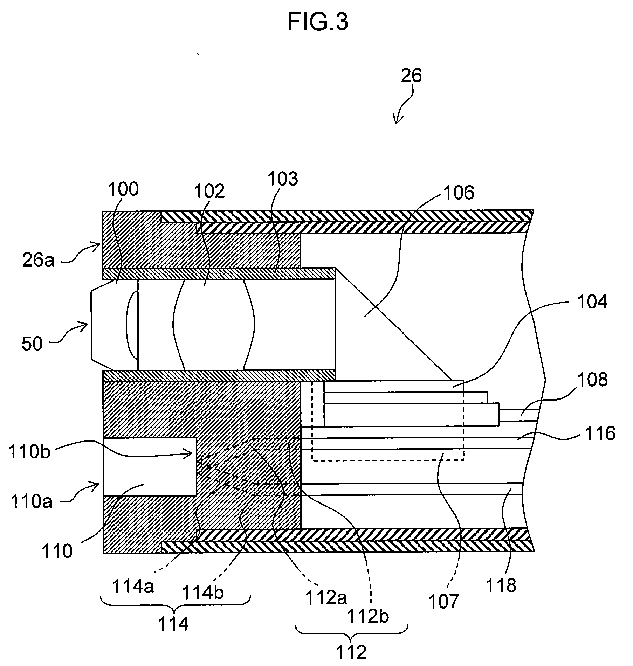

The nozzle 58 is provided so as to protrude from the distal end surface 26a of the distal end part 26, and includes the ejection port facing the observation window 50. An end part of the nozzle 58 opposite to the ejection port is connected to a confluence pipe (not illustrated in Fig. 2; illustrated in Fig. 3 by reference numeral 110) formed inside of the insertion part 20 illustrated in Fig. 1.

The confluence pipe is communicated with the connection part 30 illustrated in Fig. 1 via a gas supply pipe line (not illustrated in Fig. 2; illustrated in Fig. 3 by reference numeral 112), a liquid supply pipe line (not illustrated in Fig. 2; illustrated in Fig. 3 by reference numeral 114), a gas supply tube (not illustrated in Fig. 2; illustrated in Fig. 3 by reference numeral 116), and a liquid supply tube (not illustrated in Fig. 2; illustrated in Fig. 3 by reference numeral 118).

Further, a gas supply and liquid supply unit 40 is provided separately from the endoscope 1, and the gas supply tube and the liquid supply tube are connected to the gas supply and liquid supply unit 40 via the gas supply and liquid supply connector 34c of the connection part 30.

When the gas supply and liquid supply button 16 provided in the operation part 10 illustrated in Fig. 1 is operated, air or water (cleaning fluid) is selectively fed from the gas supply and liquid supply unit 40 via the gas supply tube, the liquid supply tube, the gas supply pipe line, and the liquid supply pipe line.

Then, the air or water fed from the gas supply and liquid supply unit 40 is ejected from the ejection port of the nozzle 58 toward the observation window 50. At the time of cleaning the observation window 50, first, water is ejected from the nozzle 58, and then, air is ejected. In this way, first, the observation window 50 is cleaned with the water, and after the cleaning, water drops remaining on the observation window 50 can be blown away by the air to be removed.

### (Description of Internal Structure of Distal end part)

Fig. 3 is a cross sectional view illustrating an internal structure of the distal end part 26 (a cross sectional view taken along the cross sectional line connecting the center of the observation window 50 and the center of the nozzle 58 in Fig. 2). As illustrated in Fig. 3, the observation window 50 is configured integrally with a cover glass 100, and an objective optical system 103 including an objective lens 102 and the like is placed on the inner side of the cover glass 100.

The cover glass 100 may serve as a lens which constitutes part of the objective optical system 103, and a plano-concave lens is generally used as the cover glass 100.

A solid state image pick-up element (for example, CCD (Charge Coupled Device) and CMOS (Complementary Metal Oxide Semiconductor)) 104 is disposed at an imaging position of the objective optical system 103. Reflected light of the light which illuminates the region to be observed via the illumination windows 52 and 54 (see Fig. 2) enters via the objective optical system 103, is refracted by approximately 90° by a prism 106, and enters a light receiving surface of the solid state image pick-up element 104. Then, the solid state image pick-up element 104 forms an optical image of the region to be observed on the light receiving surface.

The optical image of the region to be observed which is formed on the light receiving surface of the solid state image pick-up element 104 is converted into an electrical signal by the solid state image pick-up element 104, and is outputted to the processor apparatus 36 connected to the endoscope 1 (see Fig. 1) via a signal line 108. This electrical signal is converted into a video signal by the processor apparatus 36, and is displayed on a monitor 42 as an endoscope image.

In addition, the distal end part 26 is provided with a zoom mechanism 107 (indicated by a broken line in Fig. 3) for moving a movable lens (zoom lens) included in the objective optical system 103, and a zoom lever provided in the operation part 10 illustrated in Fig. 1 is operated, to thereby enable zoom adjustment.

The zoom mechanism 107 is placed below the objective optical system 103 in Fig. 3 (on the side of the confluence pipe 110 to be described later).

On the other hand, below the placement position of the objective optical system 103 in Fig. 3, the confluence pipe 110 which is communicated with the ejection port (see Fig. 8) of the nozzle 58 (see Fig. 2) is formed.

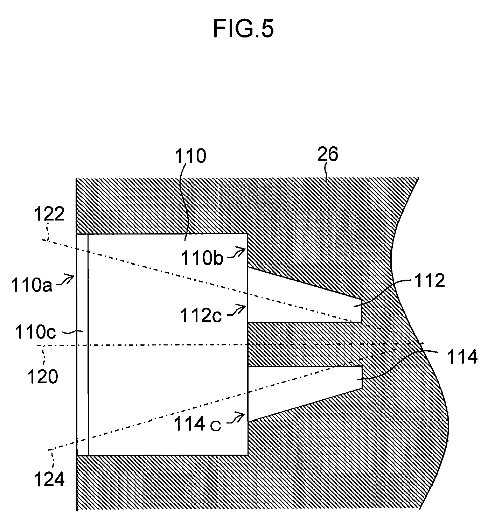

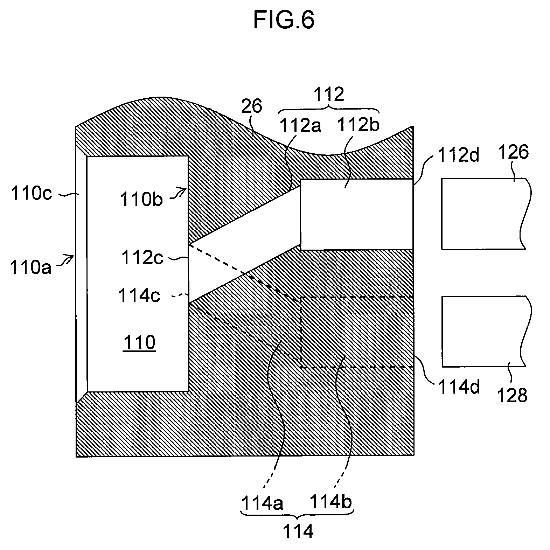

The confluence pipe 110 is formed as a concave part which extends along the central axis direction of the distal end part 26 from a substantially circular opening 110a formed on the distal end surface 26a. It should be noted that, though illustration is omitted in Fig. 3, the opening 110a of the confluence pipe 110 has been subjected to a chamfering process (illustrated in Figs. 5 and 6 by reference character 110c) corresponding to inclination of the gas supply pipe line 112 and the liquid supply pipe line 114 to be described later.

A planar surface (bottom surface) 110b on a side of an operation part (hereinafter, operation part-side planar surface 110b) of the confluence pipe 110 is connected to one end parts of the gas supply pipe line 112 and the liquid supply pipe line 114 indicated by broken lines in Fig. 3. The gas supply pipe line 112 and the liquid supply pipe line 114 are formed so as to be communicated with the confluence pipe 110 and extend from the operation part-side planar surface 110b of the confluence pipe 110 toward the operation part side (deeper side).

Another end part of the gas supply pipe line 112 is connected to the gas supply tube 116 via a gas supply pipe (not illustrated in Fig. 3; illustrated in Fig. 6 by reference numeral 126), and another end part of the liquid supply pipe line 114 is connected to the liquid supply tube 118 via a liquid supply pipe (not illustrated in Fig. 3; illustrated in Fig. 6 by reference numeral 128).

The gas supply tube 116 and the liquid supply tube 118 pass through the inside of the flexible part 22 to be communicated with the gas supply and liquid supply connector 34c.

The gas supply pipe line 112 illustrated in Fig. 3 includes: an inclined part 112a which is formed obliquely upward from a substantially central position in the top-bottom direction of the operation part-side planar surface 110b of the confluence pipe 110 (the top-bottom direction in the state where the observation window 50 is located immediately above the confluence pipe 110); and a horizontal part 112b which is formed in a direction substantially parallel to the central axis direction of the distal end part 26.

Similarly, the liquid supply pipe line 114 includes: an inclined part 114a which is formed obliquely downward from the substantially central position; and a horizontal part 114b which is formed in a direction substantially parallel to the central axis direction of the distal end part 26.

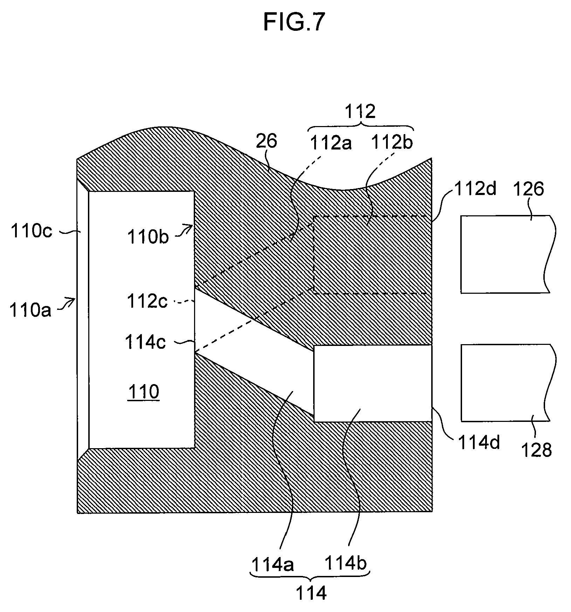

It should be noted that, in Fig. 3, for convenience of illustration, the inclined parts 112a and 114a and the horizontal parts 112b and 114b are illustrated at the same diameter, but as illustrated in Figs. 6 and 7, a part or entirety of the diameters of the horizontal parts 112b and 114b may be made larger than those of the inclined parts 112a and 114a correspondingly to the diameters of the gas supply pipe 126 and the liquid supply pipe 128 respectively connected to the horizontal parts 112b and 114b.

### (Description of Structures of Confluence Pipe, Gas Supply Pipe Line, and Liquid Supply Pipe Line)

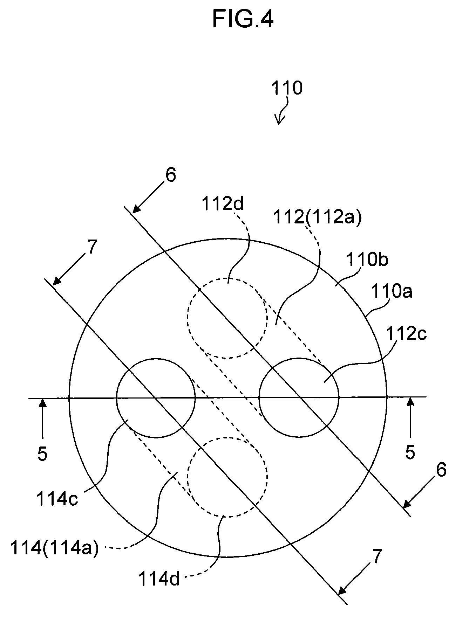

Fig. 4 is a view illustrating the operation part-side planar surface 110b of the confluence pipe 110, which is observed from the opening 110a side. In addition, Figs. 5 to 7 are cross sectional views taken along the 5-5 line, the 6-6 line, and the 7-7 line illustrated in Fig. 4, respectively.

It should be noted that the 5-5 line illustrated in Fig. 4 intersects with the 6-6 line and the 7-7 line illustrated in Fig. 4, and the 6-6 line and the 7-7 line are substantially parallel to each other.

In the state as illustrated in Fig. 4 where the observation window 50 (see Fig. 3) is located immediately above the confluence pipe 110, a hole part 112c corresponding to the one end part of the gas supply pipe line 112 and a hole part 114c corresponding to the one end part of the liquid supply pipe line 114 are placed so as to be lined in the left-right direction, and a hole part 112d corresponding to the another end part of the gas supply pipe line 112 and a hole part 114d corresponding to the another end part of the liquid supply pipe line 114 are placed so as to be lined in the top-bottom direction.

That is, the gas supply pipe line 112 is formed obliquely to the central axis direction of the distal end part 26, and is formed obliquely upward to the top-bottom direction in Fig. 3. Similarly, the liquid supply pipe line 114 is formed obliquely to the central axis direction of the distal end part 26 (in the direction opposite to the gas supply pipe line 112), and is formed obliquely downward to the top-bottom direction in Fig. 3.

The cross section along the 5-5 line (hereinafter, 5-5 cross section) of the distal end part 26 illustrated in Fig. 5 is a plane on which the hole part 112c corresponding to the one end part of the gas supply pipe line 112 and the hole part 114c corresponding to the one end part of the liquid supply pipe line 114 can be seen, the hole part 112c and the hole part 114c being formed on the operation part-side planar surface 110b of the confluence pipe 110. Further, the 5-5 cross section is a plane on which part of the one end part 112c side of the gas supply pipe line 112 and part of the one end part 114c side of the liquid supply pipe line 114 can be seen.

As illustrated in Fig. 5, at a confluence part between the confluence pipe 110 and the gas supply pipe line 112 (the one end part 112c of the gas supply pipe line 112), the direction of a central axis 120 of the confluence pipe 110 and the direction of a central axis 122 of the gas supply pipe line 112 are not parallel but at an angle to each other. At a confluence part between the confluence pipe 110 and the liquid supply pipe line 114 (the one end part 114c of the liquid supply pipe line 114), the direction of the central axis 120 of the confluence pipe 110 and the direction of a central axis 124 of the liquid supply pipe line 114 are not parallel but at an angle to each other.

In addition, the direction of the central axis 122 of the gas supply pipe line 112 at the confluence part between the confluence pipe 110 and the gas supply pipe line 112 is not parallel to the direction of the central axis 124 of the liquid supply pipe line 114 at the confluence part between the confluence pipe 110 and the liquid supply pipe line 114.

The cross section along the 6-6 line (hereinafter 6-6 cross section) illustrated in Fig. 6 is a plane on which the central axis of the inclined part 112a of the gas supply pipe line 112 exists. The 6-6 cross section is a plane on which the inclined part 112a of the gas supply pipe line 112 can be seen, the inclined part 112a being formed obliquely upward from the hole part 112c corresponding to the end part of the gas supply pipe line 112 on the confluence pipe 110 side. Further, the 6-6 cross section is a plane on which the horizontal part 112b of the gas supply pipe line 112 can be seen. That is, the 6-6 cross section is a plane on which the entire gas supply pipe line 112 can be seen. On the other hand, the 6-6 cross section is a plane on which the liquid supply pipe line 114 cannot be seen.

The cross section along the 7-7 line (hereinafter 7-7 cross section) illustrated in Fig. 7 is a plane on which the central axis of the inclined part 114a of the liquid supply pipe line 114 exists. The 7-7 cross section is a plane on which the inclined part 114a of the liquid supply pipe line 114 can be seen, the inclined part 114a being formed obliquely downward from the hole part 114c corresponding to the end part of the liquid supply pipe line 114 on the confluence pipe 110 side. Further, the 7-7 cross section is a plane on which the horizontal part 114b of the liquid supply pipe line 114 can be seen. That is, the 7-7 cross section is a plane on which the entire liquid supply pipe line 114 can be seen.

On the other hand, the 7-7 cross section illustrated in Fig. 7 is a plane on which the gas supply pipe line 112 cannot be seen. It should be noted that, as illustrated in Fig. 4, the 6-6 cross section and the 7-7 cross section are parallel to each other.

That is, the inclined part 112a of the gas supply pipe line 112 and the inclined part 114a of the liquid supply pipe line 114 are formed in a manner that: the central axis of the inclined part 112a of the gas supply pipe line 112 and the central axis of the inclined part 114a of the liquid supply pipe line 114 are not parallel to each other and exist on different planes; and the inclined part 112a of the gas supply pipe line 112 (the central axis of the inclined part 112a of the gas supply pipe line 112) and the inclined part 114a of the liquid supply pipe line 114 (the central axis of the inclined part 114a of the liquid supply pipe line 114) have a skew relationship (a so-called three-dimensional intersection relationship).

In other words, at the confluence part between the confluence pipe 110 and each of the gas supply pipe line 112 and the liquid supply pipe line 114 (operation part-side planar surface 110b), when a cross section is taken along an oblique cross sectional line (for example, the 6-6 line in Fig. 4) which is not orthogonal to the layout direction (for example, the direction of the 5-5 line in Fig. 4) of the hole part 112c corresponding to the one end part of the gas supply pipe line 112 and the hole part 114c corresponding to the one end part of the liquid supply pipe line 114, one pipe line of the gas supply pipe line 112 and the liquid supply pipe line 114 is processed so that the one pipe line can be seen whereas another pipe line thereof cannot be seen. When a cross section is taken along a cross sectional line (for example, the 7-7 line in Fig. 4) different from the above-mentioned oblique cross sectional line, the another pipe line is processed so that the another pipe line can be seen whereas the one pipe line cannot be seen.

According to distal end part for the endoscope having the above-mentioned structures of the gas supply pipe line 112 and the liquid supply pipe line 114, it is possible to rotate: the layout direction of the one end part (hole part) 112c of the gas supply pipe line 112 and the one end part (hole part) 114c of the liquid supply pipe line 114 on the operation part-side planar surface 110b of the confluence pipe 110; and the layout direction of the another end part 112d of the gas supply pipe line 112 and the another end part (hole part) 114d of the liquid supply pipe line 114, from a horizontal row to a vertical row (or from the vertical row to the horizontal row). Accordingly, it is possible to freely place the gas supply tube 116 connected to the another end part 112d of the gas supply pipe line 112 and the liquid supply tube 118 connected to the another end part 114d of the liquid supply pipe line 114.

Next, description is given of an example of joining together: the distal end part 26 including the confluence pipe 110, the gas supply pipe line 112, and the liquid supply pipe line 114 described above; the gas supply tube 116; the liquid supply tube 118; and the nozzle 58.

Fig. 8 is a see-through perspective view illustrating a schematic structure of the distal end part 26. In the distal end part 26 illustrated in Fig. 8, illustration of the image pick-up unit and the like illustrated in Fig. 3 is omitted, and a portion related to gas supply and liquid supply is mainly illustrated.

As illustrated in Fig. 8, the nozzle 58 includes: the elliptical ejection port 58a having a diameter in the long axis direction corresponding to the diameter of the observation window 50 (see Fig. 2); a first passage part 58b having one end at which the ejection port 58a is formed; and a second passage part 58c having one end communicated with another end part of the first passage part 58b, and another end with a shape corresponding to the operation part-side planar surface 110b of the confluence pipe 110.

The second passage part 58c has an outer diameter corresponding to an inner diameter of the confluence pipe 110, and is inserted into the confluence pipe 110. When the nozzle 58 having such a structure is inserted into the confluence pipe 110, the ejection port 58a of the nozzle 58 is communicated with the gas supply pipe line 112 and the liquid supply pipe line 114 via the first passage part 58b and the second passage part 58c.

The confluence pipe 110 is formed by a boring process from the distal end surface 26a side of the distal end part 26. After the formation of the confluence pipe 110, the inclined part 112a of the gas supply pipe line 112 and the inclined part 114a of the liquid supply pipe line 114 are formed by a boring process in respective oblique directions from the operation part-side planar surface 110b of the confluence pipe 110. On the other hand, the horizontal part 112b of the gas supply pipe line 112 and the horizontal part 114b of the liquid supply pipe line 114 are formed by a boring process from the operation part side.

At the time of forming the inclined parts 112a and 114a, the use of the inclination of the chamfered part 110c (see Figs. 5 to 7) formed in the opening 110a of the confluence pipe 110 enables a blade to be obliquely inserted.

That is, the chamfered part 110c is formed into a shape corresponding to the inclinations of the inclined parts 112a and 114a, whereby it is possible to avoid such a trouble that, at the time of forming the inclined parts 112a and 114a, the blade hits against an edge of the opening 110a and accordingly the boring process cannot be performed at a desired angle.

One end of the gas supply pipe 126 is joined to the horizontal part 112b of the gas supply pipe line 112, and another end of the gas supply pipe 126 opposite to the one end joined to the gas supply pipe line 112 is joined to the gas supply tube 116. Similarly, one end of the liquid supply pipe 128 is joined to the horizontal part 114b of the liquid supply pipe line 114, and another end of the liquid supply pipe 128 opposite to the one end joined to the liquid supply pipe line 114 is joined to the liquid supply tube 118.

For the gas supply tube 116 and the liquid supply tube 118, a double lumen structure in which two tubes are formed integrally with each other and are internally partitioned is preferably used.

For example, it is advisable to use tubes with the double lumen structure on the distal end side of the flexible part 22 which is bent to a large extent, and place the tubes with the double lumen structure so that a direction in which the tubes with the double lumen structure bend more easily corresponds to a bending direction of the flexible part 22. In addition, separate tubes may be used in the middle of the structure.

### (Examples of Method of Fixing Gas Supply Tube and Liquid Supply Tube)

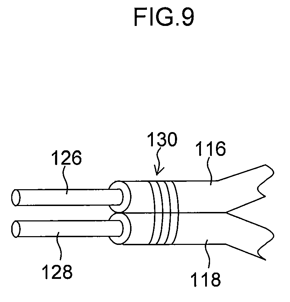

Figs. 9 to 11 each illustrates a method for fixing the gas supply tube 116 and the liquid supply tube 118. Fig. 9 illustrates a method for fixing a joining part between the gas supply pipe 126 and the gas supply tube 116 and a joining part between the liquid supply pipe 128 and the liquid supply tube 118, by string winding using a string-like fixing member 130, to thereby.

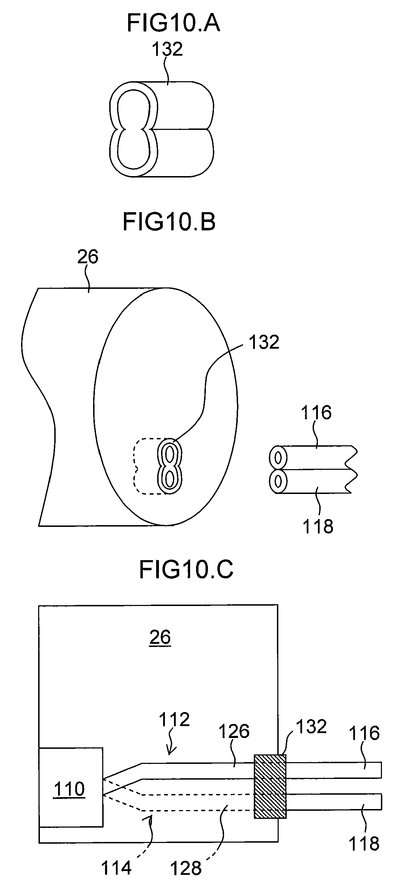

In addition, Fig. 10A is a perspective view illustrating a joining member 132 having a hollow shape corresponding to the tubes with the double lumen structure, and Fig. 10B is an explanatory view illustrating a state where the joining member 132 is attached to the gas supply pipe 126 and the liquid supply pipe 128.

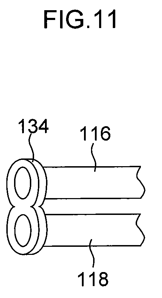

Further, Fig. 10C is an explanatory view schematically illustrating a fixing method of using the joining member 132 having the hollow shape corresponding to the tubes with the double lumen structure. Fig. 11 is an explanatory view schematically illustrating a fixing method of attaching a flange 134 to end parts of the gas supply tube 116 and the liquid supply tube 118.

It should be noted that the method illustrated in Fig. 11 of attaching the flange is also effective in using separate tubes as the gas supply tube 116 and the liquid supply tube 118.

### (Configuration Example of Bending Part)

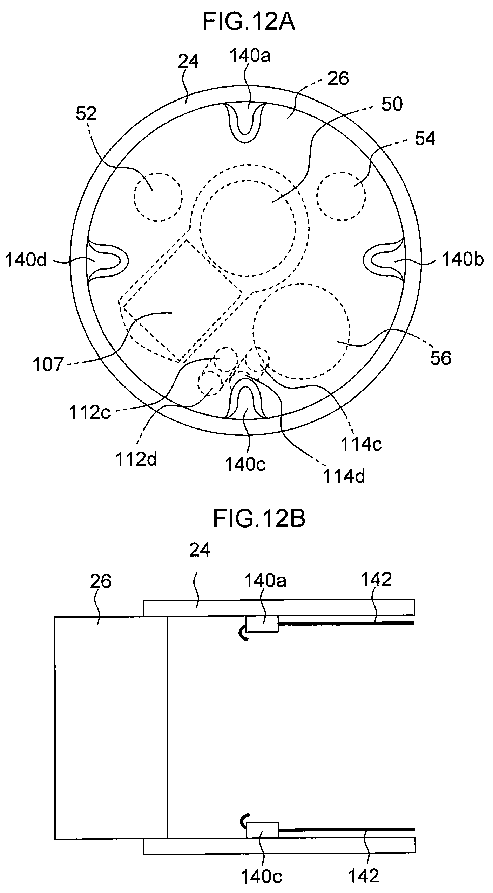

Figs. 12A and 12B are explanatory views each illustrating a schematic structure of the bending part 24 for bending the distal end of the insertion part 20 up, down, right, or left. Fig. 12A is a plan view illustrating a connection portion between the distal end part 26 and the bending part 24, which is observed from the bending part 24 side, and Fig. 12B is an explanatory view schematically illustrating a fixing structure for wires.

It should be noted that, in Fig. 12B, illustration of the signal line 108, the gas supply tube 116, the liquid supply tube 118, and the like which are placed internally is omitted.

As illustrated in Figs. 12A and 12B, in a vicinity of the connection portion between the distal end part 26 and the bending part 24, the bending part 24 is provided with four fixing parts 140a to 140d for fixing wires 142 bent into a substantially U shape.

The distal end part of the wire 142 passing in the vicinity of the inner circumferential surface of the bending part 24 is fixed by soldering or brazing to each of the fixing parts 140a to 140d. Another end part of the wire 142 is joined to the angle knob 14 (see Fig. 1) through the bending part 24 and the flexible part 22.

The bending part 24 can be bent by operating the angle knob 14 to pull any one of the four wires 142.

If the four fixing parts 140a to 140d illustrated in Fig. 12A are provided at regular intervals by 90° on the circumference constituting the inner circumference of the bending part 24, the wires 142 joined to the fixing parts 140a to 140d can be placed at regular intervals, so that an operation of bending the bending part 24 to angle the distal end part 26 (changing the orientation of the distal end part 26) can be performed in respective directions in a balanced manner.

However, if the gas supply tube 116 or the liquid supply tube 118 is placed near the wire 142 in the bending part 24 (at a position near the inner circumferential surface of the bending part), when the wire 142 is pulled, the wire 142 pushes the gas supply tube 116 or the liquid supply tube 118 inward. Therefore, in a conventional technology, the position of any one of the fixing parts 140a to 140d is shifted, whereby the position of the wire 142 is shifted so that the wire 142 does not interfere with the gas supply tube 116 or the liquid supply tube 118. In such a configuration, there is fear that an angling operation in a specific direction is not performed evenly.

The adoption of the confluence pipe 110, the gas supply pipe line 112, and the liquid supply pipe line 114 according to the present embodiment makes it possible to change as appropriate the placement of the another end part 112d of the gas supply pipe line 112 and the another end part 114d of the liquid supply pipe line 114, while the fixing parts 140a to 140d are kept at the best suited positions without shifting the positions thereof. Accordingly, the gas supply tube 116 and the liquid supply tube 118 are placed at the best suited positions, and the wire 142 can be placed at the best suited position.

According to the distal end part included in the endoscope 1 configured as described above, the gas supply pipe line 112 and the liquid supply pipe line 114 are formed in the distal end part 26 so as to have a skew positional relationship, to thereby realize the placement of the gas supply tube 116 and the liquid supply tube 118 at the best suited positions while avoiding convexoconcaves such as the image pick-up unit incorporated in the distal end part 26. In addition, this placement is compatible with the placement of the wire 142 of the bending part 24 at the best suited position.

The optimization of the fixing positions of the gas supply tube 116 and the liquid supply tube 118 in the distal end part 26 of the endoscope 1 leads to making the layouts of the gas supply tube 116 and the liquid supply tube 118 less likely to be disturbed even when being bent. As a result, an enhancement in durability of the gas supply tube 116 and the liquid supply tube 118 can be expected.

### [Modified Examples]

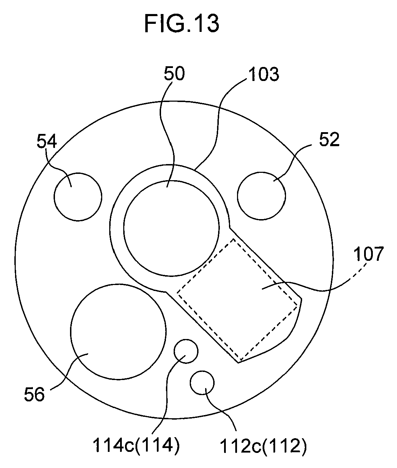

Next, with reference to Fig. 13 to Fig. 17, description is given of modified examples (a first modified example to a fifth modified example) of the structures of the confluence pipe 110, the gas supply pipe line 112, and the liquid supply pipe line 114 described above.

It should be noted that, in Fig. 13 to Fig. 17, components identical or similar to those described above are designated by the identical reference numerals or characters, and description thereof is omitted.

Fig. 13 is a plan view illustrating the distal end surface 26a of the distal end part 26 according to the first modified example. It should be noted that, in Fig. 13, the one end part (hole part) 112c of the gas supply pipe line 112 and the one end part (hole part) 114c of the liquid supply pipe line 114 are illustrated instead of the nozzle 58 illustrated in Fig. 2.

The first modified example illustrated in Fig. 13 is different from the above-mentioned embodiment in the layout direction of the one end part 112c of the gas supply pipe line 112 and the one end part 114c of the liquid supply pipe line 114 on the operation part-side planar surface 110b of the confluence pipe 110 (see Fig. 8), in order to avoid the interference (collision caused by the layout) between the zoom mechanism 107 included in the objective optical system 103 and each of the gas supply pipe line 112 and the liquid supply pipe line 114.

That is, according to the mode illustrated in Fig. 4, in the state where the observation window 50 is located immediately above the confluence pipe 110, the one end part 112c of the gas supply pipe line 112 and the one end part 114c of the liquid supply pipe line 114 are lined in the left-right direction. On the other hand, according to the mode illustrated in Fig. 13, in the same state, the one end part 112c of the gas supply pipe line 112 and the one end part 114c of the liquid supply pipe line 114 are lined (arranged) in an oblique direction.

That is, the gas supply pipe line 112 and the liquid supply pipe line 114 are formed correspondingly to the structure, size, and placement of the objective optical system 103 while avoiding the objective optical system 103. Accordingly, it is possible to change as appropriate the placement of the one end part (hole part) 112c of the gas supply pipe line 112 and the one end part (hole part) 114c of the liquid supply pipe line 114 on the operation part-side planar surface 110b of the confluence pipe 110, depending on the placement of the gas supply pipe line 112 and the liquid supply pipe line 114.

In addition, in Fig. 13, although illustration of the another end part 112d of the gas supply pipe line 112 and the another end part 114d of the liquid supply pipe line 114 is omitted, these end parts are placed as appropriate correspondingly to the objective optical system 103.

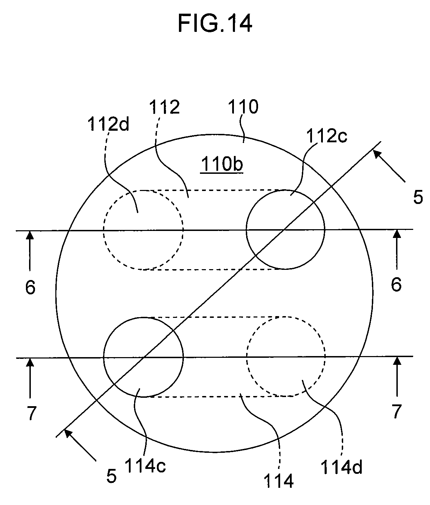

Fig. 14 is a view illustrating the placement of the gas supply pipe line 112 and the liquid supply pipe line 114 according to the second modified example. It should be noted that, in Fig. 14, the observation window 50 (see Fig. 13) is located immediately above the confluence pipe 110.

In the second modified example illustrated in Fig. 14, the gas supply pipe line 112 and the liquid supply pipe line 114 are each formed parallel to the horizontal direction in Fig. 14.

That is, the one end part 112c of the gas supply pipe line 112 and the one end part 114c of the liquid supply pipe line 114 are placed so as to be lined in an oblique direction. The gas supply pipe line 112 and the liquid supply pipe line 114 are each formed parallel to the horizontal direction in Fig. 14, and are formed obliquely to the direction (the longitudinal direction of the confluence pipe 110) of the central axis 120 (see Fig. 5) of the confluence pipe 110.

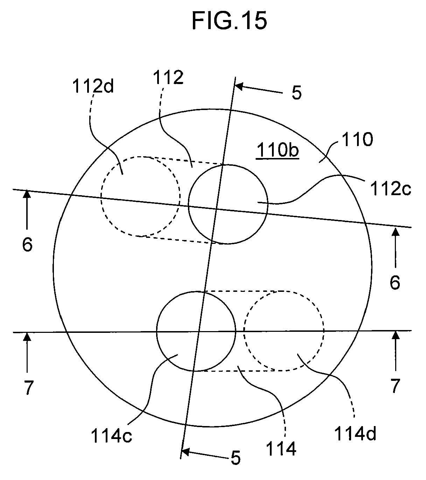

As in the third modified example illustrated in Fig. 15, the 6-6 line and the 7-7 line illustrated in Fig. 14 are not necessarily parallel to each other, the 6-6 line being a cross sectional line indicating a plane on which the central axis of the inclined part 112a of the gas supply pipe line 112 exists, the 7-7 line being a cross sectional line indicating a plane on which the central axis of the inclined part 114a of the liquid supply pipe line 114 exists.

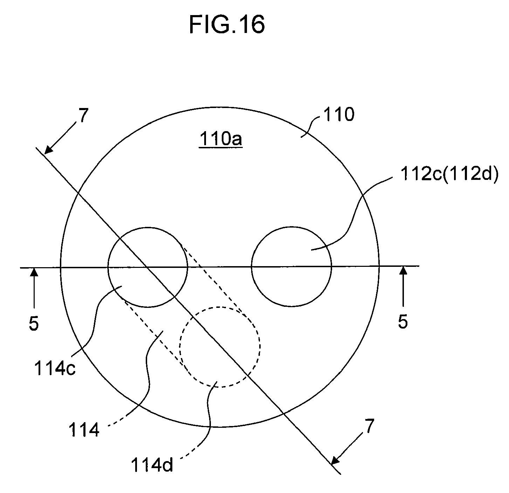

In addition, as in the fourth modified example illustrated in Fig. 16, it is possible to adopt a mode in which the 6-6 line (or the 7-7 line) does not exist, the 6-6 line being the cross sectional line indicating the plane on which the central axis of the inclined part 112a of the gas supply pipe line 112 exists, the 7-7 line being the cross sectional line indicating the plane on which the central axis of the inclined part 114a of the liquid supply pipe line 114 exists.

In the mode illustrated in Fig. 16, the gas supply pipe line 112 is formed in a direction substantially parallel to the central axis direction of the confluence pipe 110, and the liquid supply pipe line 114 is formed obliquely downward in Fig. 16.

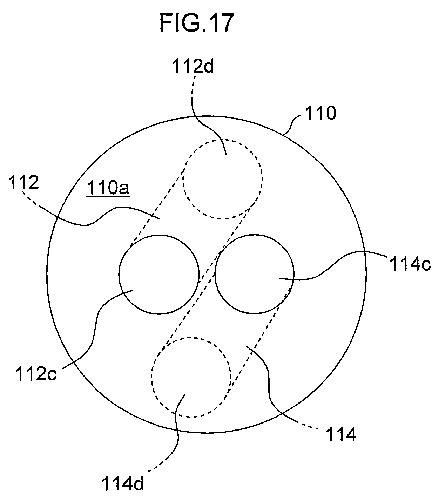

In the fifth modified example illustrated in Fig. 17, the one end (distal end side) part 112c of the gas supply pipe line 112 and the one end (distal end side) part 114c of the liquid supply pipe line 114 are placed adjacently to each other, and the another end (base end side) part 112d of the gas supply pipe line 112 and the another end (base end side) part 114d of the liquid supply pipe line 114 are placed apart from each other.

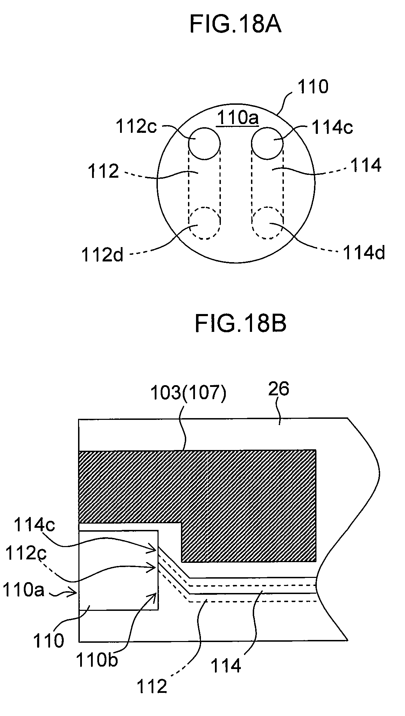

Fig. 18A is a plan view illustrating the confluence pipe 110 according to a sixth modified example, and Fig. 18B is a cross sectional view illustrating a schematic structure of the distal end part 26 according to the sixth modified example.

As illustrated in Figs. 18A and 18B, the central axis of the gas supply pipe line 112 and the central axis of the liquid supply pipe line 114 are parallel to each other, and the central axis of the gas supply pipe line 112 and the central axis of the liquid supply pipe line 114 have a skew relationship to the central axis of the confluence pipe.

As illustrated in Fig. 18B, the objective optical system 103 (zoom mechanism 107) is convex downward in the figure, and hence the gas supply pipe line 112 and the liquid supply pipe line 114 are placed so as to avoid this convex shape.

It should be noted that, in Fig. 18B, the gas supply pipe line 112 and the liquid supply pipe line 114 are illustrated so as not to overlap each other by shifting the positions thereof in the top-bottom direction. As a matter of course, as illustrated in Fig. 18B, the gas supply pipe line 112 and the liquid supply pipe line 114 may be placed with the positions thereof being shifted in the top-bottom direction.

According to the first to sixth modified examples, the hole part 112c corresponding to the one end part of the gas supply pipe line 112 and the hole part 114c corresponding to the one end part of the liquid supply pipe line 114 which are provided on the operation part-side planar surface 110b of the confluence pipe 110 can be placed at positions at which the boring process for forming the gas supply pipe line 112 and the liquid supply pipe line 114 is facilitated.

In addition, the placement of the hole part 112d corresponding to the another end part of the gas supply pipe line 112 and the hole part 114d corresponding to the another end part of the liquid supply pipe line 114 can correspond to the placement of the gas supply tube 116 and the liquid supply tube 118 which are placed correspondingly to the layout of components (such as the image pick-up unit) incorporated in the distal end part 26.

### [Second Embodiment]

Next, description is given of a distal end part for an endoscope according to a second embodiment of the presently disclosed subject matter. It should be noted that components identical or similar to those in the first embodiment described above are designated by the identical reference numerals or characters, and description thereof is omitted.

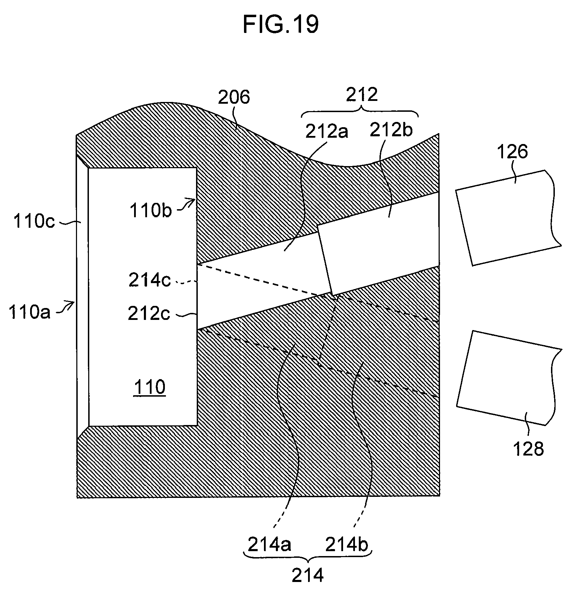

Fig. 19 is a cross sectional view illustrating a structure of a distal end part 206 for the endoscope according to the second embodiment (a view corresponding to Fig. 6 according to the first embodiment).

Unlike the gas supply pipe line 112 and the liquid supply pipe line 114 illustrated in Fig. 6, the distal end part 206 illustrated in Fig. 19 does not include bending parts (does not include the horizontal part 112b of the gas supply pipe line 112 and the horizontal part 114b of the liquid supply pipe line 114), and a gas supply pipe line 212 and a liquid supply pipe line 214 are obliquely formed in a substantially linear manner so that the gas supply pipe 126 and the liquid supply pipe 128 are obliquely inserted thereinto.

That is, the gas supply pipe line 212 illustrated in Fig. 19 includes: a small pipe part 212a which is formed obliquely upward from one end part 212c, and has substantially the same diameter as that of the one end part 212c; and a large pipe part 212b having a diameter (inner diameter) corresponding to an outer diameter of the gas supply pipe 126. The small pipe part 212a and the large pipe part 212b have the same central axis.

Similarly, the liquid supply pipe line 214 includes: a small pipe part 214a which is formed obliquely downward from one end part 214c, and has substantially the same diameter as that of the one end part 214c; and a large pipe part 214b having a diameter (inner diameter) corresponding to an outer diameter of the liquid supply pipe 128. The small pipe part 214a and the large pipe part 214b have the same central axis.

According to the present embodiment, the gas supply pipe line 212 and the liquid supply pipe line 214 are not bent but formed in the substantially linear manner, whereby the manufacturing process can be simplified. In addition, the gas supply pipe line 212 and the liquid supply pipe line 214 can be formed by only the boring process from the operation part side (in Fig. 19, the right side).

### [Third Embodiment]

Next, description is given of a distal end part for an endoscope according to a third embodiment of the presently disclosed subject matter. It should be noted that components identical or similar to those in the first and second embodiments described above are designated by the identical reference numerals or characters, and description thereof is omitted.

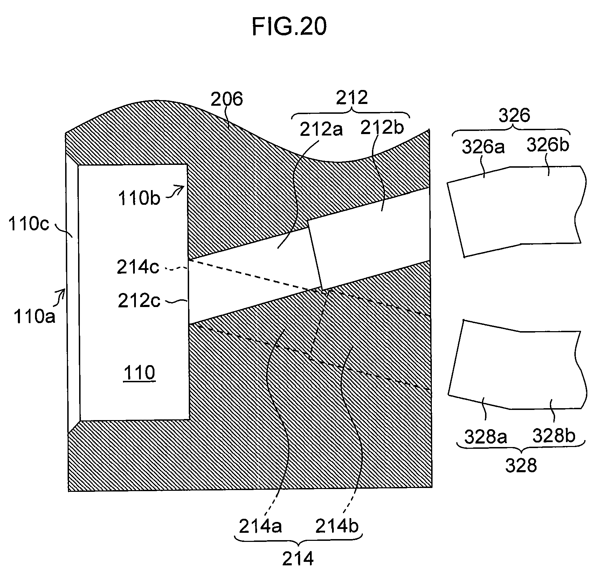

The third embodiment illustrated in Fig. 20 is different from the second embodiment illustrated in Fig. 19 in structures of a gas supply pipe 326 and a liquid supply pipe 328. On the other hand, the structure of the distal end part 206 is the same as that in the second embodiment.

That is, the gas supply pipe 326 and the liquid supply pipe 328 illustrated in Fig. 20 include: inclined pipe parts 326a and 328a which are oblique to the large pipe part 212b of the gas supply pipe line 212 and the large pipe part 214b of the liquid supply pipe line 214; and horizontal pipe parts 326b and 328b which are substantially parallel to the direction of the central axis 120 (see Fig. 5) of the confluence pipe 110, respectively.

According to the present embodiment, the take-out directions of the gas supply pipe 326 and the liquid supply pipe 328 (the joining directions of the gas supply tube 116 and the liquid supply tube 118 illustrated in Fig. 3 and the like) can be made substantially parallel to the direction of the central axis 120 of the confluence pipe 110, which makes it possible to realize the placement of the gas supply tube 116 and the liquid supply tube 118 at the best suited positions.

In addition, when the gas supply tube 116 and the gas supply pipe 326 are fixed to each other and when the liquid supply tube 118 and the liquid supply pipe 328 are fixed to each other, the bent structures of the gas supply pipe 326 and the liquid supply pipe 328 can be utilized.

### [Fourth Embodiment]

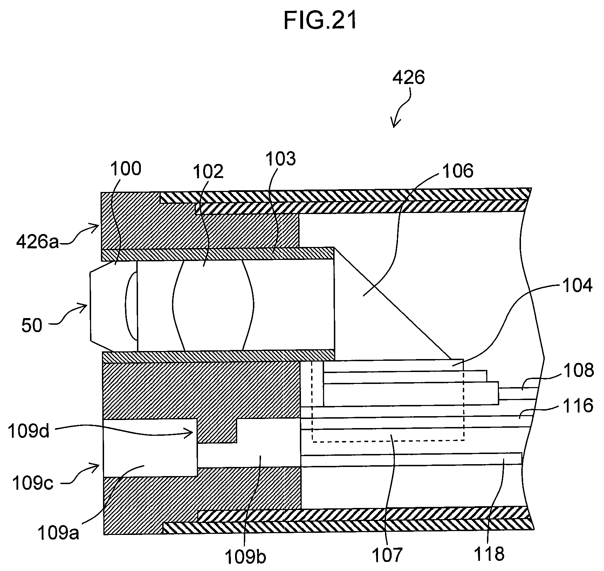

Next, a fourth embodiment not being part of the invention is described. Fig. 21 is a cross sectional view illustrating an internal structure of a distal end part 426 according to the fourth embodiment. It should be noted that, in Fig. 21, components identical or similar to those in Fig. 3 are designated by the identical reference numerals or characters, and description thereof is omitted.

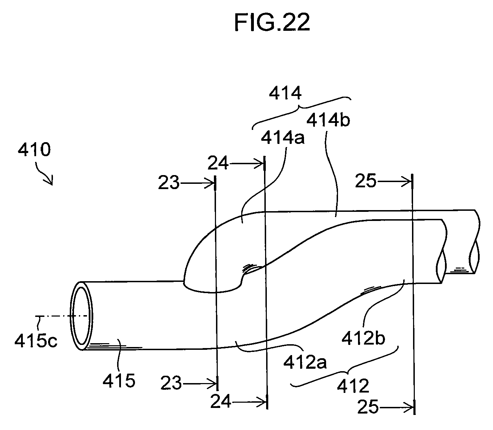

A nozzle placement part 109a on which the nozzle 58 (see Fig. 2) is placed and a confluence pipe placement part 109b on which a confluence pipe (not illustrated in Fig. 21; illustrated in Fig. 22 by reference numeral 410) is placed are formed on the central side of the distal end part 426 in Fig. 21 with respect to the placement position of the objective optical system 103.

The nozzle placement part 109a is formed as a concave part which extends in the central axis direction of the distal end part 426 from a substantially circular opening 109c formed on a distal end surface 426a.

On an operation part-side planar surface (bottom surface) 109d of the nozzle placement part 109a, the nozzle placement part 109a is communicated with the confluence pipe placement part 109b, and this structure allows the nozzle 58 placed inside of the nozzle placement part 109a to be communicated with the confluence pipe placed inside of the confluence pipe placement part 109b.

On the base end side (the side opposite to the distal end surface 426a) of the distal end part 426, the confluence pipe to be described later is connected to the gas supply tube 116 and the liquid supply tube 118. The gas supply tube 116 and the liquid supply tube 118 are communicated with the gas supply and liquid supply connector 34c (see Fig. 1) through the inside of the flexible part 22.

Fig. 22 is a perspective view illustrating a schematic structure of the confluence pipe 410. The confluence pipe 410 illustrated in Fig. 22 includes: a gas supply pipe line 412 (first communication part) communicated with the gas supply tube 116 (see Fig. 21, first fluid pipe); and a liquid supply pipe line 414 (second communication part) communicated with the liquid supply tube 118 (second fluid pipe), and further includes a confluence part 415 in which the gas supply pipe line 412 and the liquid supply pipe line 414 are brought together.

The gas supply pipe line 412 includes: a bent shape part 412a having a bent shape; and a linear shape part 412b having a substantially linear shape. The liquid supply pipe line 414 includes: a bent shape part 414a having a bent shape; and a linear shape part 414b having a substantially linear shape.

That is, the gas supply pipe line 412 and the liquid supply pipe line 414 each have a bent shape with respect to the confluence part 415.

In a joining part in which the gas supply pipe line 412 and the liquid supply pipe line 414 are joined to each other (brought together), the bent shape part 414a of the liquid supply pipe line 414 is joined to the bent shape part 412a of the gas supply pipe line 412, and the liquid supply pipe line 414 is configured so that the distal end part of the bent shape part 414a comes together with the bent shape part 412a of the gas supply pipe line 412. The confluence pipe 410 having such a structure is placed inside of the confluence pipe placement part 109b illustrated in Fig. 21 with the confluence part 415 being located on the distal end side.

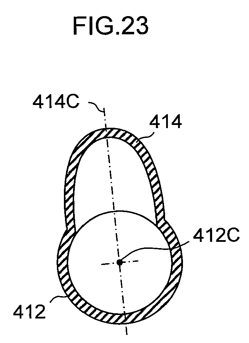

Fig. 23 is a cross sectional view taken along the 23-23 line in Fig. 22 (a cross sectional line passing through the joining part between the gas supply pipe line 412 and the liquid supply pipe line 414). As illustrated in Fig. 23, the direction of a central axis 414c of the liquid supply pipe line 414 in the joining part is a direction toward a central axis 412c of the gas supply pipe line 412.

In addition, a central axis 415c (see Fig. 22) of the confluence part 415 (not illustrated) is not parallel to any of the central axis 412c of the gas supply pipe line 412 and the central axis 414c of the liquid supply pipe line 414.

That is, the central axis 412c of the gas supply pipe line 412, the central axis 414c of the liquid supply pipe line 414, and the central axis 415c of the confluence part 415 are placed on different planes.

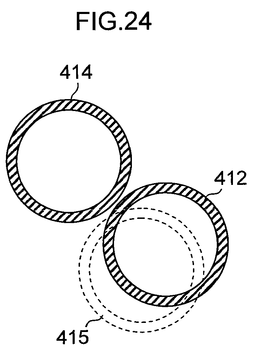

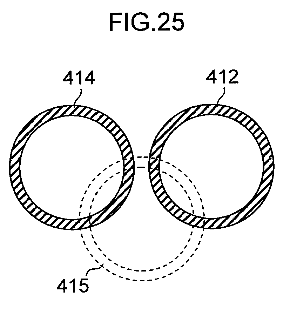

Fig. 24 is a cross sectional view taken along the 24-24 line in Fig. 22 (a cross sectional view taken in the bent shape part 412a of the gas supply pipe line 412 and the bent shape part 414a of the liquid supply pipe line 414). In addition, Fig. 25 is a cross sectional view taken along the 25-25 line in Fig. 22 (a cross sectional view taken in the linear shape part 412b of the gas supply pipe line 412 and the linear shape part 414b of the liquid supply pipe line 414).

As illustrated in Fig. 24 and Fig. 25, the gas supply pipe line 412 and the liquid supply pipe line 414 branched from the confluence part 415 are bent so that a relative positional relationship therebetween gradually changes from the distal end part side (in Fig. 22, the left side) toward the operation part side (in Fig. 22, the right side).

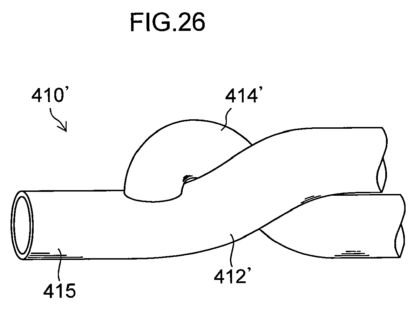

Fig. 25 illustrates a mode in which the gas supply pipe line 412 and the liquid supply pipe line 414 are placed so as to be lined in the left-right direction in Fig. 25. Alternatively, as a confluence pipe 410' illustrated in Fig. 26, a gas supply pipe line 412' and a liquid supply pipe line 414' may be configured to there-dimensionally intersect with each other and thus have a skew relationship, so that the gas supply pipe line 412' and the liquid supply pipe line 414' may be placed so as to be lined in the top-bottom direction in Fig. 25 (Fig. 24).

Still alternatively, the gas supply pipe line 412 and the liquid supply pipe line 414 may be placed so as to be lined obliquely to the left-right direction (or the top-bottom direction).

It should be noted that the confluence pipe 410 (410') which is applied to the distal end part 426 of the endoscope 1 according to the present embodiment has an inner diameter of approximately 0.8 mm to 1.0 mm and a thickness of approximately 0.1 mm to 0.15 mm.

According to the distal end part for the endoscope including the confluence pipe 410 (410') configured as described above, in the confluence pipe 410 including the gas supply pipe line 412, the liquid supply pipe line 414, and the confluence part 415, the gas supply pipe line 412 includes the bent shape part 412a having the bent shape, and the liquid supply pipe line 414 includes the bent shape part 414a having the bent shape. Further, in the joining part (confluence part) between the gas supply pipe line 412 and the liquid supply pipe line 414, the central axis 412c of the gas supply pipe line 412, the central axis 414c of the liquid supply pipe line 414, and the central axis 415c of the confluence part 415 are placed on the different planes. Accordingly, the degree of freedom in the placement of the joining part of the gas supply pipe line 412 to the gas supply tube 116 and the joining part of the liquid supply pipe line 414 to the liquid supply tube 118 is enhanced, which makes it possible to realize the placement of the gas supply tube 116 and the liquid supply tube 118 at the best suited positions.

Next, description is given of an example of joining together: the distal end part 426 including the confluence pipe 410 (410') described above; the gas supply tube 116; the liquid supply tube 118; and the nozzle 58.

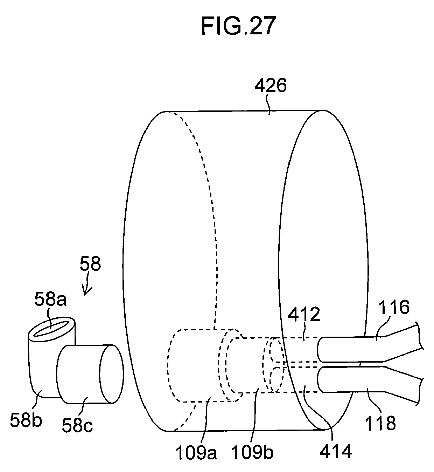

Fig. 27 is a see-through perspective view illustrating a schematic structure of the distal end part 426. In the distal end part 426 illustrated in Fig. 27, illustration of the image pick-up unit and the like illustrated in Fig. 21 is omitted, and a portion related to gas supply and liquid supply is mainly illustrated.

It should be noted that, in Fig. 27, components identical or similar to those in Fig. 8 are designated by the identical reference numerals or characters, and description thereof is omitted.

As illustrated in Fig. 27, the nozzle 58 includes: the elliptical ejection port 58a having a diameter in the long axis direction corresponding to the diameter of the observation window 50 (see Fig. 2); a first passage part 58b having one end at which the ejection port 58a is formed; and a second passage part 58c having one end communicated with another end part of the first passage part 58b, and an opposite end part coupled to the confluence part 415 (not illustrated in Fig. 27; see Fig. 22) of the confluence pipe 410.

The nozzle 58 is inserted into the nozzle placement part 109a, and the confluence pipe 410 is inserted into the confluence pipe placement part 109b. Further, at the boundary between the nozzle placement part 109a and the confluence pipe placement part 109b, the nozzle 58 and the confluence pipe 410 are coupled to each other. As a result, the nozzle 58 and the confluence pipe 410 are communicated with each other.

Moreover, the gas supply pipe line 412 and the liquid supply pipe line 414 which extend from the side of the confluence pipe placement part 109b opposite to the nozzle 58 are joined to the gas supply tube 116 and the liquid supply tube 118, respectively.

For the gas supply tube 116 and the liquid supply tube 118, the double lumen structure illustrated in Fig. 9 is preferably used. In addition, the respective methods illustrated in Figs. 10A to 10C and Fig. 11 can be applied to the method of fixing the gas supply tube and the liquid supply tube.

Similarly, the structure illustrated in Figs. 12A and 12B can be applied to the structure of the bending part. The adoption of the confluence pipe 410 according to the present embodiment makes it possible to change as appropriate the placement of the joining part of the gas supply pipe line 412 to the gas supply tube 116 and the joining part of the liquid supply pipe line 414 to the liquid supply tube 118, while the fixing parts 140a to 140d are kept at the best suited positions without shifting the positions thereof. Accordingly, the gas supply tube 116 and the liquid supply tube 118 are placed at the best suited positions, and the wire 142 can be placed at the best suited position.

The optimization of the fixing positions of the gas supply tube 116 and the liquid supply tube 118 in the distal end part 426 of the endoscope 1 leads to making the layouts of the gas supply tube 116 and the liquid supply tube 118 less likely to be disturbed even when being bent. As a result, an enhancement in durability of the gas supply tube 116 and the liquid supply tube 118 can be expected.

### [Fifth Embodiment]

Next, description is given of a gas supply and liquid supply apparatus for an endoscope according to a fifth embodiment not being part of the invention. It should be noted that components identical or similar to those in the first to fourth embodiments described above are designated by the identical reference numerals or characters, and description thereof is omitted.

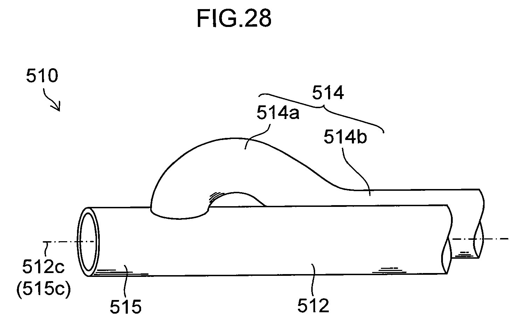

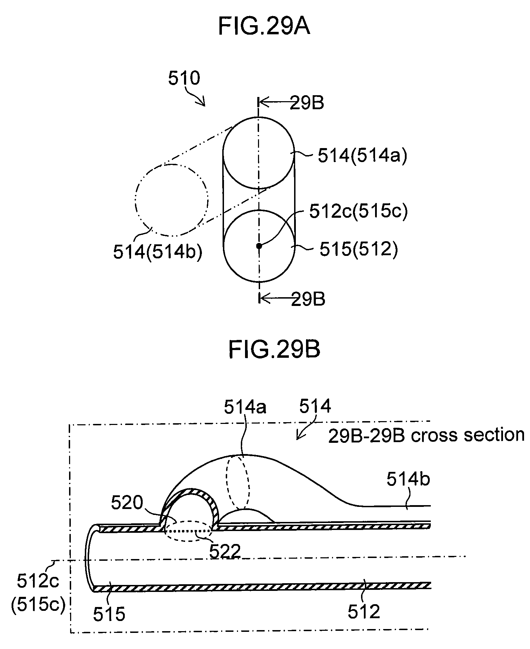

Fig. 28 is a perspective view illustrating a confluence pipe 510 included in the distal end part for the endoscope according to the fifth embodiment, and Fig. 29A is a view schematically illustrating a cross sectional shape of the confluence pipe 510 along a plane substantially orthogonal to the axial direction of a confluence part 515, which is observed from the distal end side.

In addition, Fig. 29B is a cross sectional view taken along the 29B-29B line in Fig. 29A. The confluence pipe 510 illustrated in Fig. 28 includes: a gas supply pipe line 512 which is formed in the same direction as that of the confluence part 515 and has a substantially linear shape; and a liquid supply pipe line 514 having a bent shape.

The liquid supply pipe line 514 includes: a bent shape part 514a having a shape bent from a portion joined to the gas supply pipe line 512; and a linear shape part 514b which is communicated with the bent shape part 514a and has a substantially linear shape.

That is, in the confluence pipe 510 illustrated in Fig. 28, the distal end of the bent shape part 514a of the liquid supply pipe line 514 which is bent in a direction having a circumferential component of the gas supply pipe line 512 comes together with the gas supply pipe line 512 having a central axis 512c common to a central axis 515c of the confluence part 515, and the linear shape part 514b of the liquid supply pipe line 514 is placed substantially parallel to the gas supply pipe line 512.

A circle illustrated in the lower right portion of Fig. 29A (indicated by a solid line) shows a cross sectional shape of the gas supply pipe line 512 and the confluence part 515 on the distal end side, and a circle immediately thereabove (indicated by a solid line) shows a cross sectional shape of the liquid supply pipe line 514 at a position at which the bending direction of the bent shape part 514a changes.

In addition, a circle on the left side of the confluence part 515 and the liquid supply pipe line 514 in Fig. 29A (indicated by a chain double-dashed line) shows a cross sectional shape on the base end side of the linear shape part 514b.

As illustrated in Fig. 29B, focusing on a boundary plane 520 between the gas supply pipe line 512 and the liquid supply pipe line 514, a shape obtained by projecting the outer edge of the boundary plane 520 toward the central axis 512c of the gas supply pipe line 512 is substantially elliptical (indicated by a broken line).

When the substantially elliptical shape parallel to the central axis 512c of the gas supply pipe line 512 is considered, the longest one (indicated by a thick broken line by reference numeral 522) of segments connecting two points on the outer edge of the boundary plane 520 (substantially elliptical shape) exists on the cross section along the 29B-29B line (hereinafter 29B-29B cross section).

On the other hand, the 29B-29B cross section illustrated in Fig. 29B is a plane including the central axis 512c of the gas supply pipe line 512 (the central axis 515c of the confluence part 515) and the longest segment 522 as well as a plane parallel to the central axis 512c of the gas supply pipe line 512 (the central axis 515c of the confluence part 515), and the central axis of the liquid supply pipe line 514 does not exist on the 29B-29B cross section.

Fig. 28 illustrates a mode in which an opening of the linear shape part 514b of the liquid supply pipe line 514 at an end thereof opposite to the bent shape part 514a (an opening at an end to which the liquid supply tube is joined) is lined in the left-right direction in Fig. 28 with an opening of the gas supply pipe line 512 at an end opposite to the confluence part 515 (a base end-side opening to which the gas supply tube is joined). Alternatively, the bent shape of the bent shape part 514a of the liquid supply pipe line 514 is changed, whereby the openings may be lined in the top-bottom direction in Fig. 28, or may be lined obliquely to the left-right direction in Fig. 28.

According to the distal end part for the endoscope including the confluence pipe 510 according to the fifth embodiment, it is possible to increase space behind the joining part (confluence part) between the gas supply pipe line 512 and the liquid supply pipe line 514 in the confluence pipe 510 (on the side opposite to the confluence part 515), and hence another structural component can be placed in the increased space.

In other words, the placement relationship on the side of the liquid supply pipe line 514 opposite to the confluence part 515 can be made different from the placement relationship in the joining part (confluence part) between the gas supply pipe line 512 and the liquid supply pipe line 514 with respect to the central axis 512c of the gas supply pipe line 512. Accordingly, the degree of freedom in the placement of the gas supply tube and the liquid supply tube respectively joined to the gas supply pipe line 512 and the liquid supply pipe line 514 is enhanced.

### [Sixth Embodiment]

Next, description is given of a gas supply, and liquid supply apparatus for an endoscope according to a sixth embodiment not being part of the invention. It should be noted that components identical or similar to those in the first to fifth embodiments described above are designated by the identical reference numerals or characters, and description thereof is omitted.

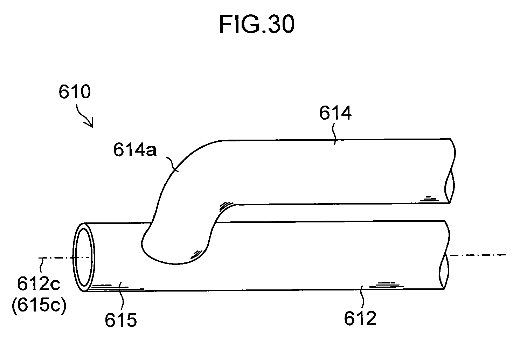

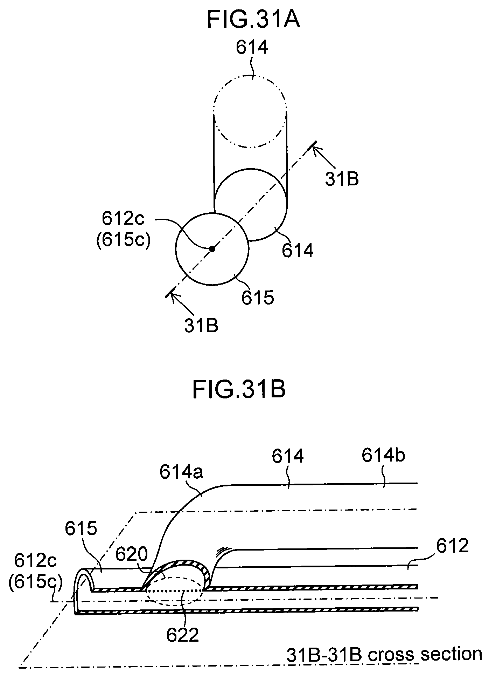

Fig. 30 is a perspective view illustrating a confluence pipe 610 included in the distal end part for the endoscope according to the sixth embodiment, and Fig. 31A is a view schematically illustrating a cross sectional shape of the confluence pipe 610, which is observed from the distal end side. In addition, Fig. 31B is a cross sectional view taken along the 31B-31B line in Fig. 31A.

The confluence pipe 610 illustrated in Fig. 30 is common to the confluence pipe 510 according to the fifth embodiment in that: a liquid supply pipe line 614 including a bent shape part 614a having a bent shape is brought together with a gas supply pipe line 612 having a substantially linear shape, and the placement relationship between an end of the gas supply pipe line 612 opposite to a confluence part 615 and an end of the liquid supply pipe line 614 opposite to the confluence part 615 is changed from the placement relationship in the joining part between the gas supply pipe line 612 and the liquid supply pipe line 614, to thereby increase space behind the joining part; and the confluence pipe 610 has a structure in which the central axis of the liquid supply pipe line 614 does not exist on the cross section along the 31B-31B line (hereinafter 31B-31B cross section) illustrated in Fig. 31B.

On the other hand, the confluence pipe 610 according to the present embodiment is different from the confluence pipe 510 according to the fifth embodiment in a joining structure between the gas supply pipe line 612 and the liquid supply pipe line 614.

That is, in the confluence pipe 610 illustrated in Fig. 30 and Figs. 31A and 31B, the liquid supply pipe line 614 is brought together obliquely to the direction of a normal to the circumferential surface of the gas supply pipe line 612.

A circle illustrated in the lower left portion of Fig. 31A by a solid line shows a cross sectional shape of the confluence part 615 on the distal end side, and a circle illustrated in the upper right portion of Fig. 31A by a solid line shows a cross sectional shape of the liquid supply pipe line 614 in a confluence part between the gas supply pipe line 612 and the liquid supply pipe line 614. In addition, a circle illustrated in Fig. 31A by a chain double-dashed line shows a cross sectional shape of the liquid supply pipe line 614 on the base end side.

As illustrated in Fig. 31B, focusing on a boundary plane 620 between the gas supply pipe line 612 and the liquid supply pipe line 614, a shape obtained by projecting the outer edge of the boundary plane 620 toward a central axis 612c of the gas supply pipe line 612 is substantially elliptical (indicated by a broken line), and is parallel to the central axis 612c of the gas supply pipe line 612, and the longest one (indicated by a thick broken line by reference numeral 622) of segments connecting two points on the outer edge of the boundary plane 620 exists on the 31B-31B cross section.

On the other hand, the 31B-31B cross section illustrated in Fig. 31B is a plane including the central axis 612c of the gas supply pipe line 612 (a central axis 615c of the confluence part 615) and the longest segment 622 as well as a plane parallel to the central axis 612c of the gas supply pipe line 612 (the central axis 615c of the confluence part 615), and the central axis of the liquid supply pipe line 614 does not exist on the 31B-31B cross section.

According to the confluence pipe 610 having such a structure, the degree of freedom in the structure of the joining part between the gas supply pipe line 612 and the liquid supply pipe line 614 can be enhanced, which contributes to the optimization of the pipe line structure of the gas supply and liquid supply pipe in the distal end part.

### [Modified Examples]

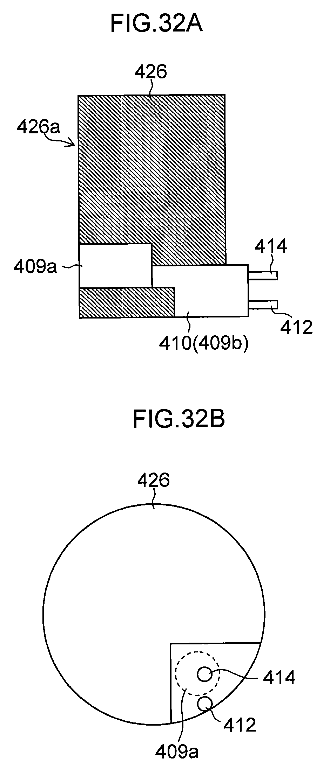

Next, a seventh modified example according to the embodiments of the presently disclosed subject matter is described. Fig. 32A is a cross sectional view schematically illustrating a structure of a distal end part 426' according to the present modified example, and Fig. 32B is a plan view of Fig. 32A, which is observed from the side opposite to the distal end surface 426a.

In the distal end part 426' according to the present modified example, the confluence pipe 410 is provided in an edge part (a lower end part in Fig. 32A). That is, a confluence pipe placement part 409b in which the confluence pipe 410 is provided is formed in a lower portion of Figs. 32A and 32B than the nozzle placement part 409a.

The adoption of the confluence pipe 410 (510, 610) according to the fourth to sixth embodiments makes it possible to place the gas supply tube and the liquid supply tube freely to some extent, and the adoption of such a structure makes it possible to place the gas supply tube and the liquid supply tube while avoiding structural components in the substantially central part of the distal end part 426 and the vicinity thereof.

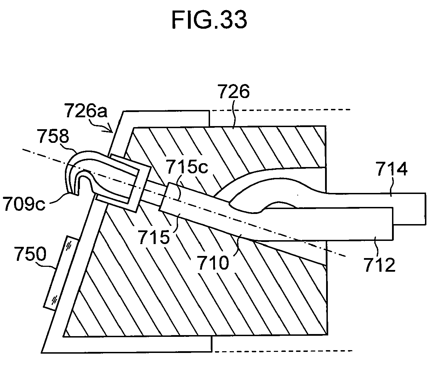

Fig. 33 is a cross sectional view illustrating a structure of a distal end part 726 according to an eighth modified example, in which illustration of part of configuration is omitted. In the distal end part 726 illustrated in Fig. 33, a distal end surface 726a is inclined, and a nozzle 758, an observation window 750, and the like are provided on the inclined distal end surface 726a.

In the distal end part 726 having such a structure, a confluence pipe 710 is inserted in a direction substantially orthogonal to the distal end surface 726a, and the confluence pipe 710 is bent in the middle thereof to become substantially parallel to the axial direction of the distal end part 726.

That is, the direction of a central axis 715c of a confluence part 715 is substantially orthogonal to the distal end surface 726a, and portions of a gas supply pipe line 712 and a liquid supply pipe line 714 on the base end side are substantially parallel to the axial direction of the distal end part 726.

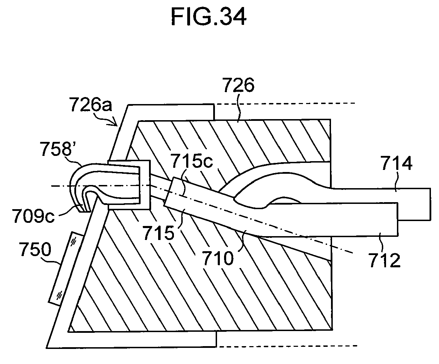

It should be noted that, as illustrated in Fig. 34, the orientation of an opening 709c of a nozzle 758' may be made oblique to the distal end surface 726a (substantially orthogonal to the central axis direction of the distal end part 726).

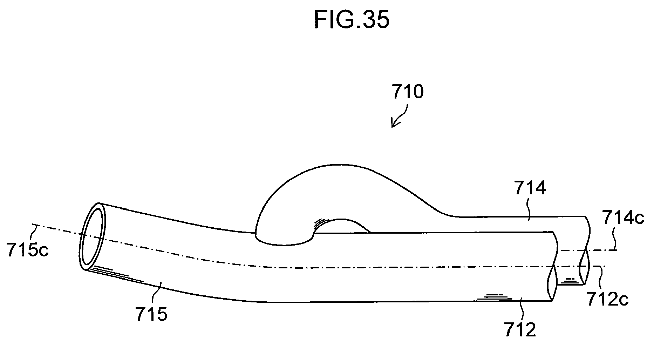

Fig. 35 is a perspective view illustrating the confluence pipe 710 illustrated in Fig. 33 and Fig. 34. The confluence pipe 710 illustrated in Fig. 35 has a structure in which: the central axis 715c of the confluence part 715 is oblique at a predetermined angle to a central axis 712c of the gas supply pipe line 712 and a central axis 714c of a base end-side portion of the liquid supply pipe line 714; and the central axis 715c of the confluence part 715, the central axis 712c of the gas supply pipe line 712, and the central axis 714c of the base end-side portion of the liquid supply pipe line 714 do not exist on the same plane.

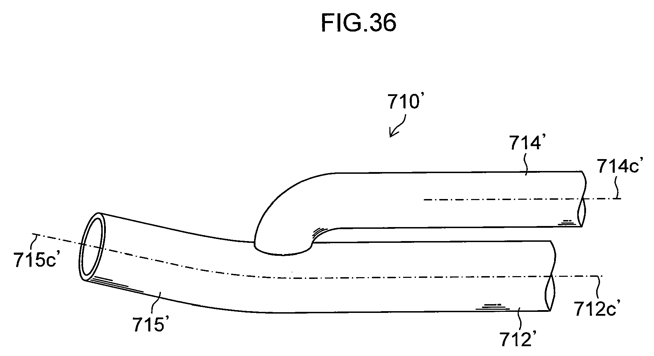

It should be noted that, as a confluence pipe 710' according to a ninth modified example illustrated in Fig. 36, a gas supply pipe line 712' and a liquid supply pipe line 714' may not be twisted.

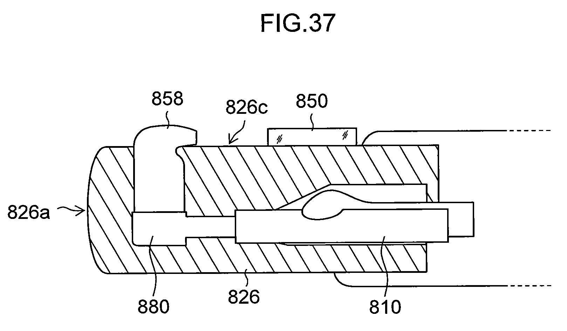

Fig. 37 is a cross sectional view schematically illustrating a structure of a distal end part 826 according to a tenth modified example. In the distal end part 826 illustrated in Fig. 37, an observation window 850, a nozzle 858, and the like are provided on a side surface 826c.

The nozzle 858 formed in a U shape, is folded back inside of the distal end part 826 to be connected to a confluence pipe 810. Fig. 37 illustrates a mode in which the confluence pipe 410 illustrated in Fig. 22 is adopted, but the confluence pipes according to all of the above-mentioned modes can be adopted.

In the distal end part 826 having such a structure, a flow channel 880 connecting the nozzle 858 and the confluence pipe 810 is inserted from the base end side of the distal end part 826, then, the nozzle 858 is inserted from the side surface 826c, and the confluence pipe 810 is inserted from the base end side.

In the first to sixth embodiments described above, description is given by mainly taking an endoscope for medical use as an example, but the presently disclosed subject matter can be applied to a bore observation apparatus for industrial use.

Hereinabove, the gas supply and liquid supply structure for the endoscope according to the presently disclosed subject matter has been described in detail, but the presently disclosed subject matter is not limited to the above-mentioned embodiments and examples and, as a matter of course, may be variously improved and modified within a range not departing from the gist of the presently disclosed subject matter.

### [Notes]

As is apparent from the embodiments of the presently disclosed subject matter described above in detail, the present specification encompasses the disclosure of a variety of technical ideas at least including the following aspects.
(Aspect 1): A gas supply and liquid supply apparatus includes: a first fluid pipe which is provided in a distal end part of an insertion part of an endoscope, the first fluid pipe for supplying a first fluid; a second fluid pipe which is provided in the distal end part of the insertion part of the endoscope together with the first fluid pipe, the second fluid pipe for supplying a second fluid; and a confluence pipe which is connected to the first fluid pipe and the second fluid pipe, a central axis of the confluence pipe in a connection portion to the first fluid pipe and the second fluid pipe having a skew relationship to at least any one of a central axis of the first fluid pipe and a central axis of the second fluid pipe.
   According to the presently disclosed subject matter, in a confluence part of the confluence pipe between the first fluid pipe and the second fluid pipe, the central axis of the confluence pipe has the skew relationship to at least any one of the central axis of the first fluid pipe and the central axis of the second fluid pipe. Accordingly, the first fluid pipe and the second fluid pipe which are connected to the confluence pipe can be three-dimensionally placed so as to suit a surrounding structure, and an enhancement in space efficiency of the portion in which the first fluid pipe and the second fluid pipe are placed can be expected.
   Specific examples of the confluence pipe according to the presently disclosed subject matter include: a mode in which the central axis of the confluence pipe in the connection part to the first fluid pipe and the second fluid pipe and the central axis of the first fluid pipe have a skew relationship; a mode in which the central axis of the confluence pipe in the connection part to the first fluid pipe and the second fluid pipe and the central axis of the second fluid pipe have a skew relationship; and a mode in which the central axis of the confluence pipe in the connection part to the first fluid pipe and the second fluid pipe and both of the central axis of the first fluid pipe and the central axis of the second fluid pipe have a skew relationship.
   The "skew relationship" in the presently disclosed subject matter refers to a concept including a relationship in which elements (lines) do not intersect with each other and are not parallel to each other. An example of such a skew relationship includes a state where the central axis of the first fluid pipe and the central axis of the second fluid pipe do not exist on the same plane.
   According to one aspect of the presently disclosed subject matter, the first fluid is defined as gas (for example, air or carbon dioxide gas), and the second fluid is defined as liquid (for example, water or a cleaning liquid). Further, it is preferable to adopt a mode including a switching device which makes switching whether to supply the first fluid to the confluence pipe or supply the second fluid to the confluence pipe.
(Aspect 2): In the gas supply and liquid supply apparatus according to Aspect 1, the first fluid pipe and the second fluid pipe are placed so as to have a skew positional relationship.
   An example of the "skew positional relationship" according to this aspect includes a positional relationship in which the first fluid pipe and the second fluid pipe are placed so as to three-dimensionally intersect with each other.
(Aspect 3): In the gas supply and liquid supply apparatus according to Aspect 1, the first fluid pipe and the second fluid pipe are placed so as to be parallel to each other.
(Aspect 4): In the gas supply and liquid supply apparatus according to any one of Aspects 1 to 3, a plane on which the central axis of the first fluid pipe is located and a plane on which the central axis of the second fluid pipe is located are parallel to each other.
(Aspect 5): In the gas supply and liquid supply apparatus according to any one of Aspects 1 to 3, a plane on which the central axis of the first fluid pipe is located and a plane on which the central axis of the second fluid pipe is located are non-parallel to each other.
(Aspect 6): In the gas supply and liquid supply apparatus according to any one of Aspects 1 to 5, the distal end part has a cylindrical shape with a distal end surface thereof being substantially circular, and the confluence pipe is formed as a concave part which has an opening on the distal end surface of the distal end part, and has a substantially cylindrical shape formed in a direction substantially parallel to a central axis direction of the distal end part.
   According to this aspect, the confluence pipe can be formed by performing a boring process along the central axis direction from the distal end surface of the distal end part.
(Aspect 7): In the gas supply and liquid supply apparatus according to Aspect 6, the confluence pipe includes a bottom surface on which: a first hole part corresponding to one end part of the first fluid pipe is formed; and a second hole part corresponding to one end part of the second fluid pipe is formed, and the first hole part and the second hole part are placed on the same line passing a center of the bottom surface of the confluence pipe.
   According to this aspect, the first fluid pipe and the second fluid pipe can be formed by performing a boring process from the bottom surface of the confluence pipe.
(Aspect 8): In the gas supply and liquid supply apparatus according to Aspect 6 or 7, the first fluid pipe and the second fluid pipe each include an inclined part which is formed obliquely to the central axis direction of the distal end part, and has one end part joined to the bottom surface opposed to the opening of the confluence pipe, and the inclined part of the first fluid pipe and the inclined part of the second fluid pipe are formed in opposite directions with respect to the central axis direction of the distal end part.
   According to this aspect, when the first fluid pipe is formed, the boring process is performed in an oblique direction from the bottom surface of the confluence pipe, and when the second fluid pipe is formed, the boring process is performed in a direction opposite to that when the first fluid pipe is formed.
(Aspect 9): In the gas supply and liquid supply apparatus according to any one of Aspects 6 to 8, the first fluid pipe and the second fluid pipe each include a horizontal part which is communicated with another end part of the inclined part, and is formed in a direction substantially parallel to the central axis direction of the distal end part.
   According to this aspect, the take-out directions of the first fluid pipe and the second fluid pipe are made substantially parallel to the central axis direction of the distal end part, whereby a pipe or a tube to be joined to the first fluid pipe and the second fluid pipe is joined more easily.
(Aspect 10): In the gas supply and liquid supply apparatus according to Aspect 9, the horizontal part of the first fluid pipe has an opposite end part to the end part communicated with the inclined part, the opposite end part being joined to a first fluid supply pipe, and the horizontal part of the second fluid pipe has an opposite end part to the end part communicated with the inclined part, the opposite end part being joined to the second fluid supply pipe.
(Aspect 11): In the gas supply and liquid supply apparatus according to any one of Aspects 4 to 8, the inclined part of the first fluid pipe has another end part joined to a first fluid supply pipe, and the inclined part of the second fluid pipe has another end part joined to a second fluid supply pipe.
(Aspect 12): In the gas supply and liquid supply apparatus according to Aspect 11, the first fluid supply pipe and the second fluid supply pipe each have a bent structure including: an inclined pipe part having inclination corresponding to the inclined part; and a horizontal pipe part extending in a direction substantially parallel to the central axis direction of the distal end part.

## Claims

1. A distal end part (26) for an insertion part (20) of an endoscope (1), wherein the endoscope comprises a nozzle (58), and the distal end part (26) comprises:
a first fluid pipe (112) which is adapted to be provided in the distal end part (26) of the insertion part (20) of the endoscope (1), the first fluid pipe (112) for supplying a first fluid;
a second fluid pipe (114) which is adapted to be provided in the distal end part (26) of the insertion part (20) of the endoscope (1) together with the first fluid pipe (112), the second fluid pipe (114) for supplying a second fluid; and
a confluence pipe (110) which is connected to the first fluid pipe (112) and the second fluid pipe (114) thereby defining two connections,
such that in a planar surface (110b) on a proximal side of the confluence pipe (110), the position of the first connection of the confluence pipe (110) to the first fluid pipe (112) differs from the position of the second connection of the confluence pipe (110) to the second fluid pipe (114),
**characterised by**
a central axis (120) of the confluence pipe (110) directed to the nozzle (58), when the distal end part (26) is positioned in the insertion part (20) of the endoscope (1), having a skew relationship to at least any one of a central axis (122) of the first fluid pipe (112) and a central axis (124) of the second fluid pipe (114) in the distal end part (26), wherein the skew relationship is a relationship in which said central axes do not intersect with each other and are not parallel to each other.

2. The distal end part (26) according to claim 1, wherein the first fluid pipe (112) and the second fluid pipe (114) are placed to have a skew positional relationship.

3. The distal end part (26) according to claim 1, wherein the first fluid pipe (112) and the second fluid pipe (114) are placed to be parallel to each other.

4. The distal end part (26) according to any one of claims 1 to 3, wherein a plane on which the central axis of the first fluid pipe (112) is located and a plane on which the central axis of the second fluid pipe (114) is located are parallel to each other.

5. The distal end part (26) according to any one of claims 1 to 3, wherein a plane on which the central axis of the first fluid pipe (112) is located and a plane on which the central axis of the second fluid pipe (114) is located are non-parallel to each other.

6. The distal end part (26) according to any one of claims 1 to 5, wherein:
the distal end part (26) has a cylindrical shape with a distal end surface (26a) thereof being substantially circular; and
the confluence pipe (110) is formed as a concave part which has an opening on the distal end surface (26a) of the distal end part (26) and has a substantially cylindrical shape formed in a direction substantially parallel to a central axis direction of the distal end part (26).

7. The distal end part (26) according to claim 6, wherein:
the confluence pipe (110) includes a bottom surface on which: a first hole part (112c) corresponding to one end part of the first fluid pipe (112) is formed; and a second hole part (114c) corresponding to one end part of the second fluid pipe (114) is formed; and
the first hole part (112c) and the second hole part (114c) are placed on a line passing a center of the bottom surface of the confluence pipe (110).

8. The distal end part (26) according to claim 6 or 7, wherein:
the first fluid pipe (112) and the second fluid pipe (114) each include an inclined part (112a, 114a) which is formed obliquely to the central axis direction of the distal end part (26), and has one end part joined to the bottom surface opposed to the opening of the confluence pipe (110); and
the inclined part (112a) of the first fluid pipe (112) and the inclined part (114a) of the second fluid pipe (114) are formed in opposite directions with respect to a direction of a central axis of the distal end part (26).

9. The distal end part (26) according to any one of claims 6 to 8, wherein the first fluid pipe (112) and the second fluid pipe (114) each include a horizontal part (112b, 114b) which is communicated with another end part of the inclined part (112a, 114a), and is formed in a direction substantially parallel to the central axis direction of the distal end part (26).

10. The distal end part (26) according to claim 9, wherein:
the horizontal part (112b) of the first fluid pipe (112) has an opposite end part to the end part communicated with the inclined part (112a), the opposite end part being joined to a first fluid supply pipe(126); and
the horizontal part (114b) of the second fluid pipe (114) has an opposite end part to the end part communicated with the inclined part (114a), the opposite end part being joined to a second fluid supply pipe (128).

11. The distal end part (26) according to any one of claims 4 to 8, wherein:
the inclined part (112a) of the first fluid pipe (112) has another end part joined to a first fluid supply pipe (126); and
the inclined part (114a) of the second fluid pipe (114) has another end part joined to a second fluid supply pipe (128).

12. The distal end part (26) according to claim 11, wherein the first fluid supply pipe (126, 326) and the second fluid supply pipe (128, 328) each have a bent structure including: an inclined pipe part (326a, 328a) having inclination corresponding to the inclined part; and a horizontal pipe part (326b, 328b)extending in a direction substantially parallel to the central axis direction of the distal end part (26, 206).

## Patentansprüche

1. Distales Endstück (26) für ein Einführstück (20) eines Endoskops (1), wobei das Endoskop eine Düse (58) umfasst und wobei das distale Endstück (26) umfasst:
eine erste Fluidleitung (112), die eingerichtet ist in dem distalen Endstück (26) des Einführstücks (20) des Endoskops (1) vorgesehen zu sein, wobei die erste Fluidleitung (112) zum Zuführen eines ersten Fluids vorgesehen ist;
eine zweite Fluidleitung (114), die eingerichtet ist in dem distalen Endstück (26) des Einführstücks (20) des Endoskops (1) zusammen mit der ersten Fluidleitung (112) vorgesehen zu sein, wobei die zweite Fluidleitung (114) zum Zuführen eines zweiten Fluids vorgesehen ist; und
eine Konfluenzleitung (110), die mit der ersten Fluidleitung (112) und der zweiten Fluidleitung (114) verbunden ist, wodurch zwei Verbindungen definiert werden,
sodass sich in einer planaren Oberfläche (110b) auf einer proximalen Seite der Konfluenzleitung (110) die Position der ersten Verbindung der Konfluenzleitung (110) zu der ersten Fluidleitung (112) von der Position der zweiten Verbindung der Konfluenzleitung (110) zu der zweiten Fluidleitung (114) unterscheidet,
**gekennzeichnet durch**
eine zu der Düse (58) gerichteten Zentralachse (120) der Konfluenzleitung (110), die, wenn das distale Endstück (26) in dem Einführstück (20) des Endoskops (1) positioniert ist, in dem distalen Endstück (26) eine windschiefe Relation zu entweder einer Zentralachse (122) der ersten Fluidleitung (112) und/oder einer Zentralachse (124) der zweiten Fluidleitung (114) aufweist, wobei die windschiefe Relation eine Relation ist, in welcher die genannten Zentralachsen miteinander nicht schneiden und zueinander nicht parallel liegen.

2. Distales Endstück (26) nach Anspruch 1, wobei die erste Fluidleitung (112) und die zweite Fluidleitung (114) so plaziert sind, dass sie eine windschiefe Positions-Relation aufweisen.

3. Distales Endstück (26) nach Anspruch 1, wobei die erste Fluidleitung (112) und die zweite Fluidleitung (114) so plaziert sind, dass sie zueinander parallel liegen.

4. Distales Endstück (26) nach einem der Ansprüche 1 bis 3, wobei eine Ebene, auf der sich die Zentralachse der ersten Fluidleitung (112) befindet und eine Ebene auf welcher sich die Zentralachse der zweiten Fluidleitung (114) befindet, zueinander parallel liegen.

5. Distales Endstück (26) nach einem der Ansprüche 1 bis 3, wobei eine Ebene, auf der sich die Zentralachse der ersten Fluidleitung (112) befindet und eine Ebene auf welcher sich die Zentralachse der zweiten Fluidleitung (114) befindet, zueinander nicht parallel liegen.

6. Distales Endstück (26) nach einem der Ansprüche 1 bis 5, wobei
das distale Endstück (26) eine zylindrische Form aufweist mit einer distalen Endoberfläche (26a), die im Wesentlich zirkular ist; und
die Konfluenzleitung (110) als ein konkaves Stück ausgebildet ist, das eine Öffnung auf der distalen Endoberfläche (26a) des distalen Endstücks (26) aufweist und eine im Wesentlichen zylindrische Form aufweist, die in einer im Wesentlichen parallel zur einer Zentralachsenrichtung des distalen Endstücks (26) liegenden Richtung ausgebildet ist.

7. Distales Endstück (26) nach Anspruch 6, wobei:
die Konfluenzleitung (110) eine Bodenoberfläche beinhaltet, auf welcher: ein erstes Lochstück (112c), das mit einem Endstück der ersten Fluidleitung (112) korrespondiert, ausgebildet ist und ein zweites Lochstück (114c), das mit einem Endstück der zweiten Fluidleitung (114) korrespondiert, ausgebildet ist; und
das erste Lochstück (112c) und das zweite Lochstück (114c) auf einer Linie plaziert sind, die durch ein Zentrum der Bodenoberfläche der Konfluenzleitung (110) verläuft.

8. Distales Endstück (26) nach Anspruch 6 oder 7, wobei:
die erste Fluidleitung (112) und die zweite Fluidleitung (114) jeweils ein geneigtes Stück (112a, 114a) beinhalten, das schräg zu der Zentralachsenrichtung des distalen Endstücks (26) ausgebildet ist und ein Endstück aufweist, das mit der Bodenoberfläche gegenüber der Öffnung der Konfluenzleitung (110) verbunden ist; und
das geneigte Stück (112a) der ersten Fluidleitung (112) und das geneigte Stück (114a) der zweiten Fluidleitung (114), bezüglich einer Richtung einer Zentralachse des distalen Endstücks (26), in gegenüberliegenden Richtungen ausgebildet sind.

9. Distales Endstück (26) nach einem der Ansprüche 6 bis 8, wobei die erste Fluidleitung (112) und die zweite Fluidleitung (114) jeweils ein horizontales Stück (112b, 114b) beinhalten, das mit einem anderen Endstück des geneigten Stücks (112a, 114a) in Verbindung steht und in einer im Wesentlichen zu der Zentralachsenrichtung des distalen Endstücks (26) parallelen Richtung ausgebildet ist.

10. Distales Endstück (26) nach Anspruch 9, wobei:
das horizontale Stück (112b) der ersten Fluidleitung (112) ein dem mit dem geneigten Stück (112a) in Verbindung stehenden Endstück gegenüberliegendes Endstück aufweist, wobei das gegenüberliegende Endstück mit einer ersten Fluidzuführleitung (126) verbunden ist; und
das horizontale Stück (114b) der zweiten Fluidleitung (114) ein dem mit dem geneigten Stück (114a) in Verbindung stehenden Endstück gegenüberliegendes Endstück aufweist, wobei das gegenüberliegende Endstück mit einer zweiten Fluidzuführleitung (128) verbunden ist.

11. Distales Enstück (26) nach einem der Ansprüche 4 bis 8, wobei:
das geneigte Stück (112a) der ersten Fluidleitung (112) ein weiteres Endstück aufweist, das mit einer ersten Fluidzuführleitung (126) verbunden ist; und
das geneigte Stück (114a) der zweiten Fluidleitung (114) ein weiteres Endstück aufweist, das mit einer zweiten Fluidzuführleitung (128) verbunden ist.

12. Distales Endstück (26) nach Anspruch 11, wobei die erste Fluidzuführleitung (126, 326) und die zweite Fluidzuführleitung (128, 328) jeweils eine gebogene Struktur aufweisen, umfassend: ein geneigtes Leitungsstück (326a, 328a) mit einer Neigung, die mit dem geneigten Stück korrespondiert; und ein horizontales Leitungsstück (326b, 328b) das sich in eine im Wesentlichen zu der Zentralachsenrichtung des distalen Endstücks (26, 206) parallelen Richtung erstreckt.

## Revendications

1. Partie d'extrémité distale (26) pour une partie d'introduction (20) d'un endoscope (1), dans laquelle l'endoscope comprend une buse (58), et la partie d'extrémité distale (26) comprend :
un premier tuyau de fluide (112), apte à être fourni dans la partie d'extrémité distale (26) de la partie d'introduction (20) de l'endoscope (1), le premier tuyau de fluide (112) étant destiné à alimenter un premier fluide ;
un second tuyau de fluide (114), apte à être fourni dans la partie d'extrémité distale (26) de la partie d'introduction (20) de l'endoscope (1) conjointement au premier tuyau de fluide (112), le second tuyau de fluide (114) étant destiné à alimenter un second fluide ;
un tuyau de confluence (110) relié au premier tuyau de fluide (112) et le second tuyau de fluide (114) définissant ainsi deux liaisons,
de sorte que dans une surface planaire (110b) sur un côté proximal du tuyau de confluence (110), la position de la première liaison du tuyau de confluence (110) avec le premier tuyau de fluide (112) diffère de la position de la seconde liaison du tuyau de confluence (110) avec le second tuyau de fluide (114),
**caractérisée par**
un axe central (120) du tuyau de confluence (110) dirigé vers la buse (58), lorsque la partie d'extrémité distale (26) est positionnée dans la partie d'introduction (20) de l'endoscope (1), présentant une relation d'obliquité avec au moins l'un quelconque d'un axe central (122) du premier tuyau de fluide (112) et d'un axe central (124) du second tuyau de fluide (114) dans la partie d'extrémité distale (26),
dans laquelle la relation d'obliquité est une relation suivant laquelle lesdits axes centraux ne se coupent pas entre eux et ne sont pas parallèles entre eux.

2. Partie d'extrémité distale (26) selon la revendication 1, dans laquelle le premier tuyau de fluide (112) et le second tuyau de fluide (114) sont placés de manière à présenter une relation de position d'obliquité.

3. Partie d'extrémité distale (26) selon la revendication 1, dans laquelle le premier tuyau de fluide (112) et le second tuyau de fluide (114) sont placés de manière à être parallèles l'un à l'autre.

4. Partie d'extrémité distale (26) selon l'une quelconque des revendications 1 à 3, dans laquelle un plan sur lequel se trouve l'axe central du premier tuyau de fluide (112) et un plan sur lequel se trouve l'axe central du second tuyau de fluide (114) sont parallèles l'un à l'autre.

5. Partie d'extrémité distale (26) selon l'une quelconque des revendications 1 à 3, dans laquelle un plan sur lequel se trouve l'axe central du premier tuyau de fluide (112) et un plan sur lequel se trouve l'axe central du second tuyau de fluide (114) ne sont pas parallèles l'un à l'autre.

6. Partie d'extrémité distale (26) selon l'une quelconque des revendications 1 à 5, dans laquelle :
la partie d'extrémité distale (26) présente une forme cylindrique, une surface d'extrémité distale (26a) de celle-ci étant en grande partie circulaire, et
le tuyau de confluence (110) est formé comme une partie concave qui présente une ouverture sur la surface d'extrémité distale (26a) de la partie d'extrémité distale (26) et présente une forme en grande partie cylindrique formée dans une direction en grande partie parallèle à une direction d'axe central de la partie d'extrémité distale (26).

7. Partie d'extrémité distale (26) selon la revendication 6, dans laquelle :
le tuyau de confluence (110) inclut une surface de fond sur laquelle : une première partie de trou (112c) correspondant à une partie d'extrémité du premier tuyau de fluide (112) est formée, et une seconde partie de trou (114c) correspondant à une partie d'extrémité du second tuyau de fluide (114) est formée, et
la première partie de trou (112c) et la seconde partie de trou (114c) sont placées sur une ligne passant par un centre de la surface de fond du tuyau de confluence (110).

8. Partie d'extrémité distale (26) selon la revendication 6 ou 7, dans laquelle :
le premier tuyau de fluide (112) et le second tuyau de fluide (114) incluent chacun une partie inclinée (112a, 114a), formée de manière oblique par rapport à la direction d'axe central de la partie d'extrémité distale (26), et présente une partie d'extrémité jointe à la surface de fond opposée à l'ouverture du tuyau de confluence (110), et
la partie inclinée (112a) du premier tuyau de fluide (112) et la partie inclinée (114a) du second tuyau de fluide (114) sont formées dans des directions opposées par rapport à une direction d'un axe central de la partie d'extrémité distale (26).

9. Partie d'extrémité distale (26) selon l'une quelconque des revendications 6 à 8, dans laquelle le premier tuyau de fluide (112) et le second tuyau de fluide (114) incluent chacun une partie horizontale (112b, 114b) qui communique avec une autre partie d'extrémité de la partie inclinée (112a, 114a), et qui est formée dans une direction en grande partie parallèle à la direction d'axe central de la partie d'extrémité distale (26).

10. Partie d'extrémité distale (26) selon la revendication 9, dans laquelle :
la partie horizontale (112b) du premier tuyau de fluide (112) présente une partie d'extrémité opposée à la partie d'extrémité communiquant avec la partie inclinée (112a), la partie d'extrémité opposée étant jointe à un premier tuyau d'alimentation en fluide (126), et
la partie horizontale (114b) du second tuyau de fluide (114) présente une partie d'extrémité opposée à la partie d'extrémité communiquant avec la partie inclinée (114a), la partie d'extrémité opposée étant jointe à un second tuyau d'alimentation en fluide (128).

11. Partie d'extrémité distale (26) selon l'une quelconque des revendications 4 à 8, dans laquelle :
la partie inclinée (112a) du premier tuyau de fluide (112) présente une autre partie d'extrémité jointe à un premier tuyau d'alimentation en fluide (126), et
la partie inclinée (114a) du second tuyau de fluide (114) présente une autre partie d'extrémité jointe à un second tuyau d'alimentation en fluide (128).

12. Partie d'extrémité distale (26) selon la revendication 11, dans laquelle le premier tuyau d'alimentation en fluide (126, 326) et le second tuyau d'alimentation en fluide (128, 328) présentent chacun une structure courbée incluant : une partie de tuyau inclinée (326a, 328a) présentant une inclinaison correspondant à la partie inclinée, et une partie de tuyau horizontale (326b, 328b) s'étendant dans une direction en grande partie parallèle à la direction d'axe central de la partie d'extrémité distale (26, 206).
